# EUROPEAN PATENT APPLICATION

(11) **EP 2 883 956 A1**
(43) Date of publication of application: **17.06.2015**
(21) Application number: 13828363.5
(22) Date of filing: 09.08.2013
(51) Int. Cl.: C12N 15/09, C12N 5/077, C12N 5/10, C12N 15/113, C12Q 1/02

(54) **PROMOTER OF DIFFERENTIATION FROM HEPATIC PROGENITOR CELL INTO HEPATIC CELL, AND USE THEREOF**

(30) Priority: 09.08.2012 JP 2012177171; 11.01.2013 JP 2013003365
(71) Applicant: National Cancer Center, Tokyo 104-0045 (JP); DS Pharma Biomedical Co., Ltd., Suita-shi, Osaka 564-0053 (JP)
(72) Inventor: OCHIYA, Takahiro, Tokyo 104-0045 (JP); GAILHOUSTE, Luc, Tokyo 104-0045 (JP)
(74) Representative: Campbell, Patrick John Henry
(86) International application number: PCT/JP2013/071726
(87) International publication number: WO 2014/025046

(57) **Abstract**

The present invention provides a promoter of differentiation from a hepatic progenitor cell into a hepatocyte, which contains a substance that suppresses expression of Dnmt-1 or a substance that inhibits the function of Dnmt-1 as an active ingredient, and a method of producing a hepatocyte (preferably hepatocyte with high maturity) from a hepatic progenitor cell.

SEQUENCE LISTING

<110>  National Cancer Center
       DS Pharma Biomedical Co., Ltd.

<120>  An Agent for Inducing Differentiation of Hepatic Progenitor Cells
        into Hepatocyte

<130>  092050

<150>  JP 2012-177171
<151>  2012-08-09

<150>  JP 2013-003365
<151>  2013-01-11

<160>  12

<170>  PatentIn version 3.3

<210>  1
<211>  5425
<212>  DNA
<213>  Homo sapiens


<220>
<221>  CDS
<222>  (181)..(5079)

<400>  1
ggctccgttc catccttctg cacagggtat cgcctctctc cgtttggtac atcccctcct      60
cccccacgcc cggactgggg tggtagacgc cgcctccgct catcgcccct ccccatcggt     120
ttccgcgcga aaagccgggg cgcctgcgct gccgccgccg cgtctgctga agcctccgag     180
atg ccg gcg cgt acc gcc cca gcc cgg gtg ccc aca ctg gcc gtc ccg       228
gcc atc tcg ctg ccc gac gat gtc cgc agg cgg ctc aaa gat ttg gaa       276
aga gac agc tta aca gaa aag gaa tgt gtg aag gag aaa ttg aat ctc       324
ttg cac gaa ttt ctg caa aca gaa ata aag aat cag tta tgt gac ttg       372
gaa acc aaa tta cgt aaa gaa gaa tta tcc gag gag ggc tac ctg gct       420
aaa gtc aaa tcc ctt tta aat aaa gat ttg tcc ttg gag aac ggt gct       468
cat gct tac aac cgg gaa gtg aat gga cgt cta gaa aac ggg aac caa       516
gca aga agt gaa gcc cgt aga gtg gga atg gca gat gcc aac agc ccc      564
ccc aaa ccc ctt tcc aaa cct cgc acg ccc agg agg agc aag tcc gat      612
gga gag gct aag cgt tca aga gac cct cct gcc tca gcc tcc caa gta      660
act ggg att aga gct gaa cct tca cct agc ccc agg att aca agg aaa      708
agc acc agg caa acc acc atc aca tct cat ttt gca aag ggc cct gcc      756
aaa cgg aaa cct cag gaa gag tct gaa aga gcc aaa tcg gat gag tcc      804
atc aag gaa gaa gac aaa gac cag gat gag aag aga cgt aga gtt aca      852
tcc aga gaa cga gtt gct aga ccg ctt cct gca gaa gaa cct gaa aga      900
gca aaa tca gga acg cgc act gaa aag gaa gaa gaa aga gat gaa aaa      948
gaa gaa aag aga ctc cga agt caa acc aaa gaa cca aca ccc aaa cag      996
aaa ctg aag gag gag ccg gac aga gaa gcc agg gca ggc gtg cag gct     1044
gac gag gac gaa gat gga gac gag aaa gat gag aag aag cac aga agt     1092
caa ccc aaa gat cta gct gcc aaa cgg agg ccc gaa gaa aaa gaa cct     1140
gaa aaa gta aat cca cag att tct gat gaa aaa gac gag gat gaa aag     1188
gag gag aag aga cgc aaa acg acc ccc aaa gaa cca acg gag aaa aaa     1236
atg gct cgc gcc aaa aca gtc atg aac tcc aag acc cac cct ccc aag     1284
tgc att cag tgc ggg cag tac ctg gac gac cct gac ctc aaa tat ggg      1332
cag cac cca cca gac gcg gtg gat gag cca cag atg ctg aca aat gag      1380
aag ctg tcc atc ttt gat gcc aac gag tct ggc ttt gag agt tat gag      1428
gcg ctt ccc cag cac aaa ctg acc tgc ttc agt gtg tac tgt aag cac      1476
ggt cac ctg tgt ccc atc gac acc ggc ctc atc gag aag aat atc gaa      1524
ctc ttc ttt tct ggt tca gca aaa cca atc tat gat gat gac cca tct      1572
ctt gaa ggt ggt gtt aat ggc aaa aat ctt ggc ccc ata aat gaa tgg      1620
tgg atc act ggc ttt gat gga ggt gaa aag gcc ctc atc ggc ttc agc      1668
acc tca ttt gcc gaa tac att ctg atg gat ccc agt ccc gag tat gcg      1716
ccc ata ttt ggg ctg atg cag gag aag atc tac atc agc aag att gtg      1764
gtg gag ttc ctg cag agc aat tcc gac tcg acc tat gag gac ctg atc      1812
aac aag atc gag acc acg gtt cct cct tct ggc ctc aac ttg aac cgc      1860
ttc aca gag gac tcc ctc ctg cga cac gcg cag ttt gtg gtg gag cag      1908
gtg gag agt tat gac gag gcc ggg gac agt gat gag cag ccc atc ttc      1956
ctg aca ccc tgc atg cgg gac ctg atc aag ctg gct ggg gtc acg ctg      2004
gga cag agg cga gcc cag gcg agg cgg cag acc atc agg cat tct acc 2052
agg gag aag gac agg gga ccc acg aaa gcc acc acc acc aag ctg gtc 2100
tac cag atc ttc gat act ttc ttc gca gag caa att gaa aag gat gac 2148
aga gaa gac aag gag aac gcc ttt aag cgc cgg cga tgt ggc gtc tgt 2196
gag gtg tgt cag cag cct gag tgt ggg aaa tgt aaa gcc tgc aag gac 2244
atg gtt aaa ttt ggt ggc agt gga cgg agc aag cag gct tgc caa gag 2292
cgg agg tgt ccc aat atg gcc atg aag gag gca gat gac gat gag gaa 2340
gtc gat gat aac atc cca gag atg ccg tca ccc aaa aaa atg cac cag 2388
ggg aag aag aag aaa cag aac aag aat cgc atc tct tgg gtc gga gaa 2436
gcc gtc aag act gat ggg aag aag agt tac tat aag aag gtg tgc att 2484
gat gcg gaa acc ctg gaa gtg ggg gac tgt gtc tct gtt att cca gat 2532
gat tcc tca aaa ccg ctg tat cta gca agg gtc acg gcg ctg tgg gag 2580
gac agc agc aac ggg cag atg ttt cac gcc cac tgg ttc tgc gct ggg 2628
aca gac aca gtc ctc ggg gcc acg tcg gac cct ctg gag ctg ttc ttg 2676
gtg gat gaa tgt gag gac atg cag ctt tca tat atc cac agc aaa gtg 2724
aaa gtc atc tac aaa gcc ccc tcc gaa aac tgg gcc atg gag gga ggc 2772
atg gat ccc gag tcc ctg ctg gag ggg gac gac ggg aag acc tac ttc 2820
tac cag ctg tgg tat gat caa gac tac gcg aga ttc gag tcc cct cca 2868
aaa acc cag cca aca gag gac aac aag ttc aaa ttc tgt gtg agc tgt 2916
gcc cgt ctg gct gag atg agg caa aaa gaa atc ccc agg gtc ctg gag 2964
cag ctc gag gac ctg gat agc cgg gtc ctc tac tac tca gcc acc aag 3012
aac ggc atc ctg tac cga gtt ggt gat ggt gtg tac ctg ccc cct gag 3060
gcc ttc acg ttc aac atc aag ctg tcc agt ccc gtg aaa cgc cca cgg 3108
aag gag ccc gtg gat gag gac ctg tac cca gag cac tac cgg aaa tac 3156
tcc gac tac atc aaa ggc agc aac ctg gat gcc cct gag ccc tac cga 3204
att ggc cgg atc aaa gag atc ttc tgt ccc aag aag agc aac ggc 3249
agg ccc aat gag act gac atc aaa atc cgg gtc aac aag ttc tac 3294
agg cct gag aac acc cac aag tcc act cca gcg agc tac cac gca 3339
gac atc aac ctg ctc tac tgg agc gac gag gag gcc gtg gtg gac 3384
ttc aag gct gtg cag ggc cgc tgc acc gtg gag tat ggg gag gac 3429
ctg ccc gag tgc gtc cag gtg tac tcc atg ggc ggc ccc aac cgc 3474
ttc tac ttc ctc gag gcc tat aat gca aag agc aaa agc ttt gaa 3519
gat cct ccc aac cat gcc cgt agc cct gga aac aaa ggg aag ggc      3564
aag gga aaa ggg aag ggc aag ccc aag tcc caa gcc tgt gag ccg      3609
agc gag cca gag ata gag atc aag ctg ccc aag ctg cgg acc ctg      3654
gat gtg ttt tct ggc tgc ggg ggg ttg tcg gag gga ttc cac caa      3699
gca ggc atc tct gac acg ctg tgg gcc atc gag atg tgg gac cct      3744
gcg gcc cag gcg ttc cgg ctg aac aac ccc ggc tcc aca gtg ttc      3789
aca gag gac tgc aac atc ctg ctg aag ctg gtc atg gct ggg gag      3834
acc acc aac tcc cgc ggc cag cgg ctg ccc cag aag gga gac gtg      3879
gag atg ctg tgc ggc ggg ccg ccc tgc cag ggc ttc agc ggc atg      3924
aac cgc ttc aat tcg cgc acc tac tcc aag ttc aaa aac tct ctg      3969
gtg gtt tcc ttc ctc agc tac tgc gac tac tac cgg ccc cgg ttc      4014
ttc ctc ctg gag aat gtc agg aac ttt gtc tcc ttc aag cgc tcc      4059
atg gtc ctg aag ctc acc ctc cgc tgc ctg gtc cgc atg ggc tat      4104
cag tgc acc ttc ggc gtg ctg cag gcc ggt cag tac ggc gtg gcc      4149
cag act agg agg cgg gcc atc atc ctg gcc gcg gcc cct gga gag      4194
aag ctc cct ctg ttc ccg gag cca ctg cac gtg ttt gct ccc cgg      4239
gcc tgc cag ctg agc gtg gtg gtg gat gac aag aag ttt gtg agc 4284
aac ata acc agg ttg agc tcg ggt cct ttc cgg acc atc acg gtg 4329
cga gac acg atg tcc gac ctg ccg gag gtg cgg aat gga gcc tcg 4374
gca ctg gag atc tcc tac aac ggg gag cct cag tcc tgg ttc cag 4419
agg cag ctc cgg ggc gca cag tac cag ccc atc ctc agg gac cac 4464
atc tgt aag gac atg agt gca ttg gtg gct gcc cgc atg cgg cac 4509
atc ccc ttg gcc cca ggg tca gac tgg cgc gat ctg ccc aac atc 4554
gag gtg cgg ctc tca gac ggc acc atg gcc agg aag ctg cgg tat 4599
acc cac cat gac agg aag aac ggc cgc agc agc tct ggg gcc ctc 4644
cgt ggg gtc tgc tcc tgc gtg gaa gcc ggc aaa gcc tgc gac ccc 4689
gca gcc agg cag ttc aac acc ctc atc ccc tgg tgc ctg ccc cac 4734
acc ggg aac cgg cac aac cac tgg gct ggc ctc tat gga agg ctc 4779
gag tgg gac ggc ttc ttc agc aca acc gtc acc aac ccc gag ccc 4824
atg ggc aag cag ggc cgc gtg ctc cac cca gag cag cac cgt gtg 4869
gtg agc gtg cgg gag tgt gcc cgc tcc cag ggc ttc cct gac acc 4914
tac cgg ctc ttc ggc aac atc ctg gac aag cac cgg cag gtg ggc 4959
aat gcc gtg cca ccg ccc ctg gcc aaa gcc att ggc ttg gag atc 5004
aag ctt tgt atg ttg gcc aaa gcc cga gag agt gcc tca gct aaa 5049
ata aag gag gag gaa gct gct aag gac tag ttctgccctc ccgtcacccc 5099
tgtttctggc accaggaatc cccaacatgc actgatgttg tgtttttaac atgtcaatct 5159
gtccgttcac atgtgtggta catggtgttt gtggccttgg ctgacatgaa gctgttgtgt 5219
gaggttcgct tatcaactaa tgatttagtg atcaaattgt gcagtacttt gtgcattctg 5279
gattttaaaa gttttttatt atgcattata tcaaatctac cactgtatga gtggaaatta 5339
agactttatg tagtttttat atgttgtaat atttcttcaa ataaatctct cctataaacc 5399
aaaaaaaaaa aaaaaaaaaa aaaaaa 5425

<210> 2
<211> 1632
<212> PRT
<213> Homo sapiens

<400> 2

<210>  3
<211>  5367
<212>  DNA
<213>  Mus musculus

<220>
<221>  CDS
<222>  (193)..(5052)

<400>  3
tagccaggag gtgtgggtgc ctccgttgcg cgcatgcgca ctcccttcgg gcatagcatg      60

gtcttccccc actctcttgc cctgtgtggt acatgctgct tccgcttgcg ccgccccctc     120

ccaattggtt tccgcgcgcg cgaaaaagcc ggggtctcgt tcagagctgt tctgtcgtct     180

gcaacctgca ag atg cca gcg cga aca gct cca gcc cga gtg cct gcg ctt     231
              Met Pro Ala Arg Thr Ala Pro Ala Arg Val Pro Ala Leu
              1               5                   10

gcc tcc ccg gca ggc tcg ctc ccg gac cat gtc cgc agg cgg ctc aaa     279
Ala Ser Pro Ala Gly Ser Leu Pro Asp His Val Arg Arg Arg Leu Lys
    15                  20                  25

gac ttg gaa aga gat ggc tta aca gaa aag gag tgt gtg agg gag aaa     327
Asp Leu Glu Arg Asp Gly Leu Thr Glu Lys Glu Cys Val Arg Glu Lys
30                  35                  40                  45

tta aac tta ctg cat gaa ttc ctg caa aca gaa ata aaa agc cag ttg     375
Leu Asn Leu Leu His Glu Phe Leu Gln Thr Glu Ile Lys Ser Gln Leu
                50                  55                  60

tgt gac ttg gaa acc aaa tta cat aaa gag gaa tta tct gag gaa ggc     423
Cys Asp Leu Glu Thr Lys Leu His Lys Glu Glu Leu Ser Glu Glu Gly
            65                  70                  75

tac ctg gct aaa gtc aag tcc ctc tta aat aag gat ttg tcc ttg gag     471
Tyr Leu Ala Lys Val Lys Ser Leu Leu Asn Lys Asp Leu Ser Leu Glu
        80                  85                  90

aac gga aca cac act ctc act caa aaa gcc aac ggt tgt ccc gcc aac     519
Asn Gly Thr His Thr Leu Thr Gln Lys Ala Asn Gly Cys Pro Ala Asn
    95                  100                 105

ggg agc cgg cca acc tgg aga gca gaa atg gca gac tca aat aga tcc     567
Gly Ser Arg Pro Thr Trp Arg Ala Glu Met Ala Asp Ser Asn Arg Ser
110                 115                 120                 125

cca aga tcc agg ccc aag cct cgg gga ccc agg aga agc aag tcg gac     615
Pro Arg Ser Arg Pro Lys Pro Arg Gly Pro Arg Arg Ser Lys Ser Asp
                130                 135                 140

agt gac acc ctt ttt gaa act tca cct agt tcc gtg gct acg agg aga     663
Ser Asp Thr Leu Phe Glu Thr Ser Pro Ser Ser Val Ala Thr Arg Arg
            145                 150                 155

acc acc agg cag acc acc atc acg gct cac ttc acg aag ggc ccc act     711
Thr Thr Arg Gln Thr Thr Ile Thr Ala His Phe Thr Lys Gly Pro Thr
        160                 165                 170

aaa cgg aaa ccc aag gaa gag tcg gaa gag ggg aac tcg gct gag tcg     759
Lys Arg Lys Pro Lys Glu Glu Ser Glu Glu Gly Asn Ser Ala Glu Ser
    175                 180                 185

gct gca gag gag aga gac cag gat aag aaa cgc aga gtt gta gac aca     807
Ala Ala Glu Glu Arg Asp Gln Asp Lys Lys Arg Arg Val Val Asp Thr
190                 195                 200                 205

gag agt ggt gct gca gct gct gtg gag aaa ctg gaa gag gta aca gcg     855
Glu Ser Gly Ala Ala Ala Ala Val Glu Lys Leu Glu Glu Val Thr Ala
                210                 215                 220

gga acc cag ctg ggt ccg gaa gag cca tgt gaa cag gaa gat gac aac     903
Gly Thr Gln Leu Gly Pro Glu Glu Pro Cys Glu Gln Glu Asp Asp Asn
            225                 230                 235

agg agt ctt cga cgt cac acc aga gag cta tca ttg agg cgg aaa tca     951
Arg Ser Leu Arg Arg His Thr Arg Glu Leu Ser Leu Arg Arg Lys Ser
        240                 245                 250

aag gag gat cca gac aga gaa gca aga ccg gaa act cac ttg gac gag     999
Lys Glu Asp Pro Asp Arg Glu Ala Arg Pro Glu Thr His Leu Asp Glu
    255                 260                 265

gac gag gac gga aaa aag gat aaa aga agt tcc aga ccc agg agc cag     1047
Asp Glu Asp Gly Lys Lys Asp Lys Arg Ser Ser Arg Pro Arg Ser Gln
270                 275                 280                 285

ccc aga gat cca gct gcc aaa cgg aga ccc aag gaa gca gag cca gag     1095
Pro Arg Asp Pro Ala Ala Lys Arg Arg Pro Lys Glu Ala Glu Pro Glu
                290                 295                 300

cag gta gct cca gag act ccc gag gac aga gac gag gat gag agg gag     1143
Gln Val Ala Pro Glu Thr Pro Glu Asp Arg Asp Glu Asp Glu Arg Glu
            305                 310                 315

gag aag aga cga aaa acg aca cgt aaa aaa ctg gag tca cac acc gtt     1191
Glu Lys Arg Arg Lys Thr Thr Arg Lys Lys Leu Glu Ser His Thr Val
        320                 325                 330

ccc gtt cag agc aga tcg gag aga aaa gcc gct caa agc aaa agt gtg     1239
Pro Val Gln Ser Arg Ser Glu Arg Lys Ala Ala Gln Ser Lys Ser Val
    335                 340                 345

atc ccg aag atc aac tca cca aag tgc ccc gag tgt ggc cag cac cta     1287
Ile Pro Lys Ile Asn Ser Pro Lys Cys Pro Glu Cys Gly Gln His Leu
350                 355                 360                 365

gac gac cct aac ctg aag tac cag cag cac cct gag gat gct gtg gat     1335
Asp Asp Pro Asn Leu Lys Tyr Gln Gln His Pro Glu Asp Ala Val Asp

370                 375                 380

gaa ccc cag atg ttg acc agt gag aaa ctg tcc atc tac gac tcc acc     1383
Glu Pro Gln Met Leu Thr Ser Glu Lys Leu Ser Ile Tyr Asp Ser Thr
            385                 390                 395

tcg acc tgg ttt gat act tat gaa gat tct ccc atg cat agg ttc act     1431
Ser Thr Trp Phe Asp Thr Tyr Glu Asp Ser Pro Met His Arg Phe Thr
        400                 405                 410

tcc ttc agt gtg tac tgc agt cgc ggg cac ctg tgt cct gtc gac acc     1479
Ser Phe Ser Val Tyr Cys Ser Arg Gly His Leu Cys Pro Val Asp Thr
    415                 420                 425

ggt ctc att gag aag aat gta gag ctc tac ttt tct ggg tgt gcc aaa     1527
Gly Leu Ile Glu Lys Asn Val Glu Leu Tyr Phe Ser Gly Cys Ala Lys
430                 435                 440                 445

gca att cat gac gag aat cca tct atg gaa ggt ggt att aat ggc aaa     1575
Ala Ile His Asp Glu Asn Pro Ser Met Glu Gly Gly Ile Asn Gly Lys
                450                 455                 460

aac ctc ggg cca atc aat cag tgg tgg ctc agt ggc ttt gat ggt ggc     1623
Asn Leu Gly Pro Ile Asn Gln Trp Trp Leu Ser Gly Phe Asp Gly Gly
            465                 470                 475

gag aag gtg ctc att ggc ttc tcc act gca ttt gct gaa tac att ttg     1671
Glu Lys Val Leu Ile Gly Phe Ser Thr Ala Phe Ala Glu Tyr Ile Leu
        480                 485                 490

atg gag ccc agc aaa gag tat gag cca ata ttt ggg ctg atg cag gag     1719
Met Glu Pro Ser Lys Glu Tyr Glu Pro Ile Phe Gly Leu Met Gln Glu
    495                 500                 505

aaa att tac atc agc aag att gtt gtt gag ttc ctg caa aac aat cct     1767
Lys Ile Tyr Ile Ser Lys Ile Val Val Glu Phe Leu Gln Asn Asn Pro
510                 515                 520                 525

gat gct gta tat gaa gac ctg atc aat aag att gag acc act gtt cct     1815
Asp Ala Val Tyr Glu Asp Leu Ile Asn Lys Ile Glu Thr Thr Val Pro
                530                 535                 540

cct tct acc att aat gtg aac cgg ttc aca gag gac tcc ctc tta cgc     1863
Pro Ser Thr Ile Asn Val Asn Arg Phe Thr Glu Asp Ser Leu Leu Arg
            545                 550                 555

cac gcc cag ttt gta gtg agc cag gta gag agt tac gac gaa gcc aag     1911
His Ala Gln Phe Val Val Ser Gln Val Glu Ser Tyr Asp Glu Ala Lys
        560                 565                 570

gac gat gat gag acc ccc atc ttc ttg tct ccc tgt atg aga gcc ctg     1959
Asp Asp Asp Glu Thr Pro Ile Phe Leu Ser Pro Cys Met Arg Ala Leu
    575                 580                 585

atc cat ttg gct ggt gtc tcc ctg gga cag agg cga gca aca agg cgc     2007
Ile His Leu Ala Gly Val Ser Leu Gly Gln Arg Arg Ala Thr Arg Arg
590                 595                 600                 605

gtc atg ggt gct acc aag gag aag gac aaa gca ccc acg aaa gcc acc     2055
Val Met Gly Ala Thr Lys Glu Lys Asp Lys Ala Pro Thr Lys Ala Thr
                610                 615                 620

acc acc aag ctg gtc tat cag atc ttt gac act ttc ttc tca gag cag     2103

Thr Thr Lys Leu Val Tyr Gln Ile Phe Asp Thr Phe Phe Ser Glu Gln
                625                 630                 635

att gag aag tat gat aag gag gac aag gag aat gcc atg aag cgc cgc      2151
Ile Glu Lys Tyr Asp Lys Glu Asp Lys Glu Asn Ala Met Lys Arg Arg
            640                 645                 650

cgc tgt ggt gtc tgt gag gtc tgt cag cag cct gag tgt ggg aag tgc      2199
Arg Cys Gly Val Cys Glu Val Cys Gln Gln Pro Glu Cys Gly Lys Cys
    655                 660                 665

aag gcg tgc aaa gat atg gtg aag ttt ggt ggc act gga cgg agt aag      2247
Lys Ala Cys Lys Asp Met Val Lys Phe Gly Gly Thr Gly Arg Ser Lys
670                 675                 680                 685

cag gct tgc ctc aag agg agg tgt cct aac ttg gcg gtg aag gag gca      2295
Gln Ala Cys Leu Lys Arg Arg Cys Pro Asn Leu Ala Val Lys Glu Ala
                690                 695                 700

gac gac gat gaa gag gct gat gat gat gtg tca gag atg cca tca ccc      2343
Asp Asp Asp Glu Glu Ala Asp Asp Asp Val Ser Glu Met Pro Ser Pro
            705                 710                 715

aaa aag ctg cat cag ggg aag aag aag aag cag aac aag gac cgc atc      2391
Lys Lys Leu His Gln Gly Lys Lys Lys Lys Gln Asn Lys Asp Arg Ile
        720                 725                 730

tcc tgg ctt ggg cag cct atg aag att gaa gag aat aga act tac tat      2439
Ser Trp Leu Gly Gln Pro Met Lys Ile Glu Glu Asn Arg Thr Tyr Tyr
    735                 740                 745

cag aag gtg agc atc gat gag gag atg cta gag gtg ggc gac tgc gtc      2487
Gln Lys Val Ser Ile Asp Glu Glu Met Leu Glu Val Gly Asp Cys Val
750                 755                 760                 765

tcg gtc att cca gat gat tcc tcc aaa cca ctc tat cta gcc agg gtc      2535
Ser Val Ile Pro Asp Asp Ser Ser Lys Pro Leu Tyr Leu Ala Arg Val
                770                 775                 780

aca gct ctg tgg gaa gac aaa aat ggt cag atg atg ttc cat gcg cac      2583
Thr Ala Leu Trp Glu Asp Lys Asn Gly Gln Met Met Phe His Ala His
            785                 790                 795

tgg ttc tgc gct ggg aca gac aca gtc ctg gga gcc acc tcc gac ccc      2631
Trp Phe Cys Ala Gly Thr Asp Thr Val Leu Gly Ala Thr Ser Asp Pro
        800                 805                 810

ctg gaa ctg ttc ctg gtg ggc gag tgc gaa aac atg cag ctt tcc tac      2679
Leu Glu Leu Phe Leu Val Gly Glu Cys Glu Asn Met Gln Leu Ser Tyr
    815                 820                 825

atc cac agc aag gtc aag gtc atc tac aaa gcc cct tct gaa aac tgg      2727
Ile His Ser Lys Val Lys Val Ile Tyr Lys Ala Pro Ser Glu Asn Trp
830                 835                 840                 845

gcc atg gag gga ggc aca gac cct gag acc aca ctg cct ggg gct gag      2775
Ala Met Glu Gly Gly Thr Asp Pro Glu Thr Thr Leu Pro Gly Ala Glu
                850                 855                 860

gat ggc aag act tac ttc ttc cag ctc tgg tac aac cag gag tac gca      2823
Asp Gly Lys Thr Tyr Phe Phe Gln Leu Trp Tyr Asn Gln Glu Tyr Ala
            865                 870                 875

agg ttt gaa tcc cca ccc aag acc cag ccg acc gag gac aac aag cac     2871
Arg Phe Glu Ser Pro Pro Lys Thr Gln Pro Thr Glu Asp Asn Lys His
        880                 885                 890

aag ttc tgc cta tct tgt atc cgg ctg gct gag ctg aga caa aaa gaa     2919
Lys Phe Cys Leu Ser Cys Ile Arg Leu Ala Glu Leu Arg Gln Lys Glu
    895                 900                 905

atg ccc aag gtc ctg gaa caa att gag gag gtg gat ggc cgg gtc tac     2967
Met Pro Lys Val Leu Glu Gln Ile Glu Glu Val Asp Gly Arg Val Tyr
910                 915                 920                 925

tgc agt tcc atc acc aag aat ggt gtt gtc tac cga ctg ggt gac agt     3015
Cys Ser Ser Ile Thr Lys Asn Gly Val Val Tyr Arg Leu Gly Asp Ser
                930                 935                 940

gtg tac ctt cct ccc gag gcc ttt act ttc aac atc aaa gtg gct agc     3063
Val Tyr Leu Pro Pro Glu Ala Phe Thr Phe Asn Ile Lys Val Ala Ser
            945                 950                 955

ccc gtg aaa cgc cca aag aag gat cct gtg aac gag acc ctg tac cct     3111
Pro Val Lys Arg Pro Lys Lys Asp Pro Val Asn Glu Thr Leu Tyr Pro
        960                 965                 970

gag cac tac cgc aag tat tct gac tac atc aag ggg agc aac ctg gat     3159
Glu His Tyr Arg Lys Tyr Ser Asp Tyr Ile Lys Gly Ser Asn Leu Asp
    975                 980                 985

gct cca gag ccc tat cgc atc ggt cgg ata aaa  gag atc cac tgt ggc     3207
Ala Pro Glu Pro Tyr Arg Ile Gly Arg Ile Lys  Glu Ile His Cys Gly
990                 995                 1000                1005

aag aag aaa ggc aag  gtc aac gag gca gac  atc aag ctg agg ctc     3252
Lys Lys Lys Gly Lys  Val Asn Glu Ala Asp  Ile Lys Leu Arg Leu
                1010                1015                1020

tac aag ttc tac agg  cct gag aat acc cac  agg tcc tac aac gga     3297
Tyr Lys Phe Tyr Arg  Pro Glu Asn Thr His  Arg Ser Tyr Asn Gly
                1025                1030                1035

tcc tat cac act gac  atc aac atg ctt tac  tgg agc gac gag gaa     3342
Ser Tyr His Thr Asp  Ile Asn Met Leu Tyr  Trp Ser Asp Glu Glu
                1040                1045                1050

gct gtg gtg aac ttc  agc gac gtg cag ggc  cgc tgt acc gtg gag     3387
Ala Val Val Asn Phe  Ser Asp Val Gln Gly  Arg Cys Thr Val Glu
                1055                1060                1065

tac ggg gaa gac cta  ctt gag agc atc cag  gat tat tca caa ggg     3432
Tyr Gly Glu Asp Leu  Leu Glu Ser Ile Gln  Asp Tyr Ser Gln Gly
                1070                1075                1080

ggc cct gac cgc ttc  tac ttc ctc gag gcc  tac aat tca aag acc     3477
Gly Pro Asp Arg Phe  Tyr Phe Leu Glu Ala  Tyr Asn Ser Lys Thr
                1085                1090                1095

aag aac ttt gaa gac  cca cca aac cat gcc  cgc agc cct ggg aac     3522
Lys Asn Phe Glu Asp  Pro Pro Asn His Ala  Arg Ser Pro Gly Asn
                1100                1105                1110

aaa ggg aaa ggg aag  ggg aaa ggg aag ggg  aag ggg aag cat cag     3567
Lys Gly Lys Gly Lys  Gly Lys Gly Lys Gly  Lys Gly Lys His Gln
                1115                1120                1125

gtg tca gag ccc aaa gag cct gag gca gcc atc aaa ctg ccc aag 3612
Val Ser Glu Pro Lys Glu Pro Glu Ala Ala Ile Lys Leu Pro Lys
                1130                1135                1140

ctc cgg acc ctg gat gtg ttt tcc ggc tgt gga ggg tta tcg gaa 3657
Leu Arg Thr Leu Asp Val Phe Ser Gly Cys Gly Gly Leu Ser Glu
                1145                1150                1155

gga ttc cac caa gca ggc atc tcg gaa acg ctg tgg gcc atc gag 3702
Gly Phe His Gln Ala Gly Ile Ser Glu Thr Leu Trp Ala Ile Glu
                1160                1165                1170

atg tgg gac ccg gca gcc cag gca ttt cgg ctg aac aac ccc ggc 3747
Met Trp Asp Pro Ala Ala Gln Ala Phe Arg Leu Asn Asn Pro Gly
                1175                1180                1185

acc aca gtg ttc aca gag gac tgc aac gtg ctt ctt aag ctg gtc 3792
Thr Thr Val Phe Thr Glu Asp Cys Asn Val Leu Leu Lys Leu Val
                1190                1195                1200

atg gct ggg gag gtg acc aac tct ctg ggc caa agg ctg cca cag 3837
Met Ala Gly Glu Val Thr Asn Ser Leu Gly Gln Arg Leu Pro Gln
                1205                1210                1215

aag ggc gat gtg gag atg ctg tgt ggt ggg cca ccc tgc cag ggc 3882
Lys Gly Asp Val Glu Met Leu Cys Gly Gly Pro Pro Cys Gln Gly
                1220                1225                1230

ttc agt ggc atg aac cgc ttc aac tcc cgc act tac tcc aag ttc 3927
Phe Ser Gly Met Asn Arg Phe Asn Ser Arg Thr Tyr Ser Lys Phe
                1235                1240                1245

aaa aac tcc cta gtg gtc tcc ttc ctc agc tac tgt gac tac tac 3972
Lys Asn Ser Leu Val Val Ser Phe Leu Ser Tyr Cys Asp Tyr Tyr
                1250                1255                1260

cgg cct cgg ttc ttc ctt ctg gag aac gtc agg aac ttc gtg tcc 4017
Arg Pro Arg Phe Phe Leu Leu Glu Asn Val Arg Asn Phe Val Ser
                1265                1270                1275

tac aga cgc tcc atg gtg ctg aag ctc aca ctg cgc tgc ctg gtc 4062
Tyr Arg Arg Ser Met Val Leu Lys Leu Thr Leu Arg Cys Leu Val
                1280                1285                1290

cgc atg ggc tac cag tgc acc ttt ggt gtg ctc cag gct gga cag 4107
Arg Met Gly Tyr Gln Cys Thr Phe Gly Val Leu Gln Ala Gly Gln
                1295                1300                1305

tat ggc gtg gcc cag aca cga agg agg gcc atc atc ttg gct gca 4152
Tyr Gly Val Ala Gln Thr Arg Arg Arg Ala Ile Ile Leu Ala Ala
                1310                1315                1320

gcc cca gga gaa aag ctg cct ctg ttc cca gag cct ctg cat gtg 4197
Ala Pro Gly Glu Lys Leu Pro Leu Phe Pro Glu Pro Leu His Val
                1325                1330                1335

ttt gcg ccc cgt gcc tgc cag ctg agc gtt gtg gtg gat gac aag 4242
Phe Ala Pro Arg Ala Cys Gln Leu Ser Val Val Val Asp Asp Lys
                1340                1345                1350

aag ttt gtt agc aac ata acg agg ctg agc tcg ggg ccc ttc cga 4287
Lys Phe Val Ser Asn Ile Thr Arg Leu Ser Ser Gly Pro Phe Arg

                    1355                    1360                    1365

acc atc acc gtg cga gac acc atg tct gac ctc ccc gag atc cag        4332
Thr Ile Thr Val Arg Asp Thr Met Ser Asp Leu Pro Glu Ile Gln
                    1370                    1375                    1380

aat gga gcc tcg aat tct gag atc ccc tac aat gga gag cca ctg        4377
Asn Gly Ala Ser Asn Ser Glu Ile Pro Tyr Asn Gly Glu Pro Leu
                    1385                    1390                    1395

tcc tgg ttc cag agg cag ctg cga gga tca cac tac cag ccc atc        4422
Ser Trp Phe Gln Arg Gln Leu Arg Gly Ser His Tyr Gln Pro Ile
                    1400                    1405                    1410

ctc agg gac cat atc tgc aag gac atg agc cca ctg gtg gct gcc        4467
Leu Arg Asp His Ile Cys Lys Asp Met Ser Pro Leu Val Ala Ala
                    1415                    1420                    1425

cgc atg cgg cac atc cca ctg ttc cca gga tca gat tgg cgt gac        4512
Arg Met Arg His Ile Pro Leu Phe Pro Gly Ser Asp Trp Arg Asp
                    1430                    1435                    1440

ctg ccc aac ata cag gtg cgg ctg gga gat ggc gtc ata gcc cat        4557
Leu Pro Asn Ile Gln Val Arg Leu Gly Asp Gly Val Ile Ala His
                    1445                    1450                    1455

aag cta cag tac acc ttt cat gat gtg aaa aat ggc tac agc agc        4602
Lys Leu Gln Tyr Thr Phe His Asp Val Lys Asn Gly Tyr Ser Ser
                    1460                    1465                    1470

acc ggt gcc ctg cgt gga gtc tgt tcc tgt gca gaa ggc aag gcc        4647
Thr Gly Ala Leu Arg Gly Val Cys Ser Cys Ala Glu Gly Lys Ala
                    1475                    1480                    1485

tgc gac cct gag tcc agg caa ttc agc acc ctc atc ccc tgg tgc        4692
Cys Asp Pro Glu Ser Arg Gln Phe Ser Thr Leu Ile Pro Trp Cys
                    1490                    1495                    1500

ctg ccg cac act ggg aac cgg cac aac cac tgg gct ggc ctc tac        4737
Leu Pro His Thr Gly Asn Arg His Asn His Trp Ala Gly Leu Tyr
                    1505                    1510                    1515

ggg cgt ctg gag tgg gat ggc ttc ttc agc acc act gtc acc aac        4782
Gly Arg Leu Glu Trp Asp Gly Phe Phe Ser Thr Thr Val Thr Asn
                    1520                    1525                    1530

cct gag ccc atg ggc aag cag ggt cgg gtg ctc cac ccg gag cag        4827
Pro Glu Pro Met Gly Lys Gln Gly Arg Val Leu His Pro Glu Gln
                    1535                    1540                    1545

cac cgg gtc gtg agt gtt cgg gaa tgt gcc cgc tcc cag ggc ttt        4872
His Arg Val Val Ser Val Arg Glu Cys Ala Arg Ser Gln Gly Phe
                    1550                    1555                    1560

cca gat agc tac cgg ttc ttc ggc aac atc ctg gac aga cac cgg        4917
Pro Asp Ser Tyr Arg Phe Phe Gly Asn Ile Leu Asp Arg His Arg
                    1565                    1570                    1575

cag gtg ggt aat gct gtg cca cca ccc ctg gcc aaa gcc att ggc        4962
Gln Val Gly Asn Ala Val Pro Pro Pro Leu Ala Lys Ala Ile Gly
                    1580                    1585                    1590

ctg gag att aag ctc tgc ctg ctg tcc agt gct cgg gag agc gca        5007

Leu Glu Ile Lys Leu Cys Leu Leu Ser Ser Ala Arg Glu Ser Ala
                1595                1600                1605

tca gct gca gtt aaa gca aaa gag gag gct gct acc aag gac tag 5052
Ser Ala Ala Val Lys Ala Lys Glu Glu Ala Ala Thr Lys Asp
                1610                1615

tgctctcacc cagagcccca cgtgcactga tgtttttaac cctttgagcc ccatcatttg 5112

aagtcttgtg ctcagtgtct gtggccatgg ctgacactaa gctgtttgta tgaggtttgt 5172

tttgtgacca agctgtgtag tactttgtgc attctgaatt ttaaggtttt tttttttgtt 5232

tggtttggtt tggtttggtt tttttcttat cctgtattct atcagatctg ccactgtgca 5292

ggtggcaagt gagacttgat gtagttttat atgttgtaat atttcttcaa aataaagcgc 5352

ttctgtcaag caccc 5367

<210> 4
<211> 1619
<212> PRT
<213> Mus musculus

<400> 4

Met Pro Ala Arg Thr Ala Pro Ala Arg Val Pro Ala Leu Ala Ser Pro
1               5                   10                  15

Ala Gly Ser Leu Pro Asp His Val Arg Arg Arg Leu Lys Asp Leu Glu
            20                  25                  30

Arg Asp Gly Leu Thr Glu Lys Glu Cys Val Arg Glu Lys Leu Asn Leu
        35                  40                  45

Leu His Glu Phe Leu Gln Thr Glu Ile Lys Ser Gln Leu Cys Asp Leu
    50                  55                  60

Glu Thr Lys Leu His Lys Glu Glu Leu Ser Glu Glu Gly Tyr Leu Ala
65                  70                  75                  80

Lys Val Lys Ser Leu Leu Asn Lys Asp Leu Ser Leu Glu Asn Gly Thr
                85                  90                  95

His Thr Leu Thr Gln Lys Ala Asn Gly Cys Pro Ala Asn Gly Ser Arg
            100                 105                 110

Pro Thr Trp Arg Ala Glu Met Ala Asp Ser Asn Arg Ser Pro Arg Ser
        115                 120                 125

Arg Pro Lys Pro Arg Gly Pro Arg Arg Ser Lys Ser Asp Ser Asp Thr
    130                 135                 140

Leu Phe Glu Thr Ser Pro Ser Ser Val Ala Thr Arg Arg Thr Thr Arg
145                 150                 155                 160

Gln Thr Thr Ile Thr Ala His Phe Thr Lys Gly Pro Thr Lys Arg Lys
                165                 170                 175

Pro Lys Glu Glu Ser Glu Glu Gly Asn Ser Ala Glu Ser Ala Ala Glu
            180                 185                 190

Glu Arg Asp Gln Asp Lys Lys Arg Arg Val Val Asp Thr Glu Ser Gly
        195                 200                 205

Ala Ala Ala Ala Val Glu Lys Leu Glu Glu Val Thr Ala Gly Thr Gln
    210                 215                 220

Leu Gly Pro Glu Glu Pro Cys Glu Gln Glu Asp Asp Asn Arg Ser Leu
225                 230                 235                 240

Arg Arg His Thr Arg Glu Leu Ser Leu Arg Arg Lys Ser Lys Glu Asp
                245                 250                 255

Pro Asp Arg Glu Ala Arg Pro Glu Thr His Leu Asp Glu Asp Glu Asp
            260                 265                 270

Gly Lys Lys Asp Lys Arg Ser Ser Arg Pro Arg Ser Gln Pro Arg Asp
        275                 280                 285

Pro Ala Ala Lys Arg Arg Pro Lys Glu Ala Glu Pro Glu Gln Val Ala
    290                 295                 300

Pro Glu Thr Pro Glu Asp Arg Asp Glu Asp Glu Arg Glu Glu Lys Arg
305                 310                 315                 320

Arg Lys Thr Thr Arg Lys Lys Leu Glu Ser His Thr Val Pro Val Gln
                325                 330                 335

Ser Arg Ser Glu Arg Lys Ala Ala Gln Ser Lys Ser Val Ile Pro Lys
            340                 345                 350

Ile Asn Ser Pro Lys Cys Pro Glu Cys Gly Gln His Leu Asp Asp Pro
        355                 360                 365

Asn Leu Lys Tyr Gln Gln His Pro Glu Asp Ala Val Asp Glu Pro Gln
    370                 375                 380

Met Leu Thr Ser Glu Lys Leu Ser Ile Tyr Asp Ser Thr Ser Thr Trp
385                 390                 395                 400

Phe Asp Thr Tyr Glu Asp Ser Pro Met His Arg Phe Thr Ser Phe Ser
                405                 410                 415

Val Tyr Cys Ser Arg Gly His Leu Cys Pro Val Asp Thr Gly Leu Ile
            420                 425                 430

Glu Lys Asn Val Glu Leu Tyr Phe Ser Gly Cys Ala Lys Ala Ile His
        435                 440                 445

Asp Glu Asn Pro Ser Met Glu Gly Gly Ile Asn Gly Lys Asn Leu Gly
    450                 455                 460

Pro Ile Asn Gln Trp Trp Leu Ser Gly Phe Asp Gly Gly Glu Lys Val
465                 470                 475                 480

Leu Ile Gly Phe Ser Thr Ala Phe Ala Glu Tyr Ile Leu Met Glu Pro
                485                 490                 495

Ser Lys Glu Tyr Glu Pro Ile Phe Gly Leu Met Gln Glu Lys Ile Tyr
            500                 505                 510

Ile Ser Lys Ile Val Val Glu Phe Leu Gln Asn Asn Pro Asp Ala Val
        515                 520                 525

Tyr Glu Asp Leu Ile Asn Lys Ile Glu Thr Thr Val Pro Pro Ser Thr
    530                 535                 540

Ile Asn Val Asn Arg Phe Thr Glu Asp Ser Leu Leu Arg His Ala Gln
545                 550                 555                 560

Phe Val Val Ser Gln Val Glu Ser Tyr Asp Glu Ala Lys Asp Asp Asp
                565                 570                 575

Glu Thr Pro Ile Phe Leu Ser Pro Cys Met Arg Ala Leu Ile His Leu
            580                 585                 590

Ala Gly Val Ser Leu Gly Gln Arg Arg Ala Thr Arg Arg Val Met Gly
        595                 600                 605

Ala Thr Lys Glu Lys Asp Lys Ala Pro Thr Lys Ala Thr Thr Thr Lys
    610                 615                 620

Leu Val Tyr Gln Ile Phe Asp Thr Phe Phe Ser Glu Gln Ile Glu Lys
625                 630                 635                 640

Tyr Asp Lys Glu Asp Lys Glu Asn Ala Met Lys Arg Arg Arg Cys Gly
                645                 650                 655

Val Cys Glu Val Cys Gln Gln Pro Glu Cys Gly Lys Cys Lys Ala Cys
        660                 665                 670

Lys Asp Met Val Lys Phe Gly Gly Thr Gly Arg Ser Lys Gln Ala Cys
    675                 680                 685

Leu Lys Arg Arg Cys Pro Asn Leu Ala Val Lys Glu Ala Asp Asp Asp
    690                 695                 700

Glu Glu Ala Asp Asp Asp Val Ser Glu Met Pro Ser Pro Lys Lys Leu
705                 710                 715                 720

His Gln Gly Lys Lys Lys Lys Gln Asn Lys Asp Arg Ile Ser Trp Leu
                725                 730                 735

Gly Gln Pro Met Lys Ile Glu Glu Asn Arg Thr Tyr Tyr Gln Lys Val
            740                 745                 750

Ser Ile Asp Glu Glu Met Leu Glu Val Gly Asp Cys Val Ser Val Ile
        755                 760                 765

Pro Asp Asp Ser Ser Lys Pro Leu Tyr Leu Ala Arg Val Thr Ala Leu
    770                 775                 780

Trp Glu Asp Lys Asn Gly Gln Met Met Phe His Ala His Trp Phe Cys
785                 790                 795                 800

Ala Gly Thr Asp Thr Val Leu Gly Ala Thr Ser Asp Pro Leu Glu Leu
                805                 810                 815

Phe Leu Val Gly Glu Cys Glu Asn Met Gln Leu Ser Tyr Ile His Ser
            820                 825                 830

Lys Val Lys Val Ile Tyr Lys Ala Pro Ser Glu Asn Trp Ala Met Glu
        835                 840                 845

Gly Gly Thr Asp Pro Glu Thr Thr Leu Pro Gly Ala Glu Asp Gly Lys
    850                 855                 860

Thr Tyr Phe Phe Gln Leu Trp Tyr Asn Gln Glu Tyr Ala Arg Phe Glu
865                 870                 875                 880

Ser Pro Pro Lys Thr Gln Pro Thr Glu Asp Asn Lys His Lys Phe Cys
                885                 890                 895

Leu Ser Cys Ile Arg Leu Ala Glu Leu Arg Gln Lys Glu Met Pro Lys

900                 905                 910

Val Leu Glu Gln Ile Glu Glu Val Asp Gly Arg Val Tyr Cys Ser Ser
        915                 920                 925

Ile Thr Lys Asn Gly Val Val Tyr Arg Leu Gly Asp Ser Val Tyr Leu
    930                 935                 940

Pro Pro Glu Ala Phe Thr Phe Asn Ile Lys Val Ala Ser Pro Val Lys
945                 950                 955                 960

Arg Pro Lys Lys Asp Pro Val Asn Glu Thr Leu Tyr Pro Glu His Tyr
                965                 970                 975

Arg Lys Tyr Ser Asp Tyr Ile Lys Gly Ser Asn Leu Asp Ala Pro Glu
            980                 985                 990

Pro Tyr Arg Ile Gly Arg Ile Lys Glu Ile His Cys Gly Lys Lys Lys
        995                 1000                1005

Gly Lys Val Asn Glu Ala Asp Ile Lys Leu Arg Leu Tyr Lys Phe
    1010                1015                1020

Tyr Arg Pro Glu Asn Thr His Arg Ser Tyr Asn Gly Ser Tyr His
    1025                1030                1035

Thr Asp Ile Asn Met Leu Tyr Trp Ser Asp Glu Glu Ala Val Val
    1040                1045                1050

Asn Phe Ser Asp Val Gln Gly Arg Cys Thr Val Glu Tyr Gly Glu
    1055                1060                1065

Asp Leu Leu Glu Ser Ile Gln Asp Tyr Ser Gln Gly Gly Pro Asp
    1070                1075                1080

Arg Phe Tyr Phe Leu Glu Ala Tyr Asn Ser Lys Thr Lys Asn Phe
    1085                1090                1095

Glu Asp Pro Pro Asn His Ala Arg Ser Pro Gly Asn Lys Gly Lys
    1100                1105                1110

Gly Lys Gly Lys Gly Lys Gly Lys Gly Lys His Gln Val Ser Glu
    1115                1120                1125

Pro Lys Glu Pro Glu Ala Ala Ile Lys Leu Pro Lys Leu Arg Thr
    1130                1135                1140

Leu Asp Val Phe Ser Gly Cys Gly Gly Leu Ser Glu Gly Phe His
    1145                1150                1155

Gln Ala Gly Ile Ser Glu Thr Leu Trp Ala Ile Glu Met Trp Asp
    1160                1165                1170

Pro Ala Ala Gln Ala Phe Arg Leu Asn Asn Pro Gly Thr Thr Val
    1175                1180                1185

Phe Thr Glu Asp Cys Asn Val Leu Leu Lys Leu Val Met Ala Gly
    1190                1195                1200

Glu Val Thr Asn Ser Leu Gly Gln Arg Leu Pro Gln Lys Gly Asp
    1205                1210                1215

Val Glu Met Leu Cys Gly Gly Pro Pro Cys Gln Gly Phe Ser Gly
    1220                1225                1230

Met Asn Arg Phe Asn Ser Arg Thr Tyr Ser Lys Phe Lys Asn Ser
    1235                1240                1245

Leu Val Val Ser Phe Leu Ser Tyr Cys Asp Tyr Tyr Arg Pro Arg
    1250                1255                1260

Phe Phe Leu Leu Glu Asn Val Arg Asn Phe Val Ser Tyr Arg Arg
    1265                1270                1275

Ser Met Val Leu Lys Leu Thr Leu Arg Cys Leu Val Arg Met Gly
    1280                1285                1290

Tyr Gln Cys Thr Phe Gly Val Leu Gln Ala Gly Gln Tyr Gly Val
    1295                1300                1305

Ala Gln Thr Arg Arg Arg Ala Ile Ile Leu Ala Ala Ala Pro Gly
    1310                1315                1320

Glu Lys Leu Pro Leu Phe Pro Glu Pro Leu His Val Phe Ala Pro
    1325                1330                1335

Arg Ala Cys Gln Leu Ser Val Val Val Asp Asp Lys Lys Phe Val
    1340                1345                1350

Ser Asn Ile Thr Arg Leu Ser Ser Gly Pro Phe Arg Thr Ile Thr
    1355                1360                1365

Val Arg Asp Thr Met Ser Asp Leu Pro Glu Ile Gln Asn Gly Ala
    1370                1375                1380

Ser Asn Ser Glu Ile Pro Tyr Asn Gly Glu Pro Leu Ser Trp Phe
    1385                1390                1395

Gln Arg Gln Leu Arg Gly Ser His Tyr Gln Pro Ile Leu Arg Asp
    1400                1405                1410

His Ile Cys Lys Asp Met Ser Pro Leu Val Ala Ala Arg Met Arg
    1415                1420                1425

His Ile Pro Leu Phe Pro Gly Ser Asp Trp Arg Asp Leu Pro Asn
    1430                1435                1440

Ile Gln Val Arg Leu Gly Asp Gly Val Ile Ala His Lys Leu Gln
    1445                1450                1455

Tyr Thr Phe His Asp Val Lys Asn Gly Tyr Ser Ser Thr Gly Ala
    1460                1465                1470

Leu Arg Gly Val Cys Ser Cys Ala Glu Gly Lys Ala Cys Asp Pro
    1475                1480                1485

Glu Ser Arg Gln Phe Ser Thr Leu Ile Pro Trp Cys Leu Pro His
    1490                1495                1500

Thr Gly Asn Arg His Asn His Trp Ala Gly Leu Tyr Gly Arg Leu
    1505                1510                1515

Glu Trp Asp Gly Phe Phe Ser Thr Thr Val Thr Asn Pro Glu Pro
    1520                1525                1530

Met Gly Lys Gln Gly Arg Val Leu His Pro Glu Gln His Arg Val
    1535                1540                1545

Val Ser Val Arg Glu Cys Ala Arg Ser Gln Gly Phe Pro Asp Ser
    1550                1555                1560

Tyr Arg Phe Phe Gly Asn Ile Leu Asp Arg His Arg Gln Val Gly
    1565                1570                1575

Asn Ala Val Pro Pro Pro Leu Ala Lys Ala Ile Gly Leu Glu Ile
    1580                1585                1590

Lys Leu Cys Leu Leu Ser Ser Ala Arg Glu Ser Ala Ser Ala Ala
    1595                1600                1605

Val Lys Ala Lys Glu Glu Ala Ala Thr Lys Asp
    1610                1615

<210>  5
<211>  22
<212>  RNA
<213>  Homo sapiens

<400>  5
ucagugcacu acagaacuuu gu                                                22


<210>  6
<211>  22
<212>  RNA
<213>  Mus musculus

<400>  6
ucagugcacu acagaacuuu gu                                                22


<210>  7
<211>  68
<212>  RNA
<213>  Homo sapiens

<400>  7
gaggcaaagu ucugagacac uccgacucug aguaugauag aagucagugc acuacagaac      60

uuugucuc                                                                68


<210>  8
<211>  99
<212>  RNA
<213>  Mus musculus

<400>  8
agccaguuug gucuuuugag acaaaguucu gagacacucc gacucugagu augauagaag      60

ucagugcacu acagaacuuu gucucuagag gcugugguc                             99


<210>  9
<211>  21
<212>  DNA
<213>  Artificial

<220>
<223>  Dnmt-1 siRNA sense strand. Combined DNA/RNA.

<400>  9
gguagagagu uacgacgaat t                                                 21


<210>  10
<211>  21
<212>  DNA
<213>  Artificial

<220>
<223>  Dnmt-1 siRNA antisense strand. Combined DNA/RNA.

<400>  10

uucgucguaa cucucuacct g                                                21

<210>  11
<211>  21
<212>  DNA
<213>  Artificial

<220>
<223>  Dnmt-1 siRNA sense strand. Combined DNA/RNA.

<400>  11
caacggaucc uaucacacut t                                                21

<210>  12
<211>  21
<212>  DNA
<213>  Artificial

<220>
<223>  Dnmt-1 siRNA antisense strand. Combined DNA/RNA.

<400>  12
agugugauag gauccguugt a                                                21

## Description

### Technical Field

The present invention relates to a promoter of differentiation from hepatic progenitor cell or hepatoblastoma into hepatocyte, a method of producing hepatocyte from hepatic progenitor cell or hepatoblastoma and the like.

### Background Art

In new drug development processes, tests utilizing hepatocytes are performed to predict hepatotoxicity or liver metabolite. As hepatocytes, human primary cultured hepatocytes are generally used. However, since human primary cultured hepatocytes are associated with problems that (1) a good lot is difficult to obtain in Japan since they depend on the import from abroad, (2) since primary cells do not grow, the number of samples in the same lot is limited, and the like, which prevent stable supply and continuous use thereof. Therefore, a method of obtaining a hepatocyte by differentiation induction of a stem cell (artificial pluripotent stem cell (iPS cell), embryonic stem cell (ES cell), mesenchymal stem cell) showing superior growth has been demanded.

As a method for inducing differentiation of stem cell into hepatocyte, a method including stepwise introduction of plural genes into iPS cell (non-patent document 1), a method including culturing mesenchymal stem cells in a special differentiation induction medium (non-patent document 2) and the like are known. However, it is known that a hepatocyte prepared by differentiation induction of stem cell by a conventional method shows low drug-metabolizing enzyme activity (CYP activity) and the like as compared to human primary cultured hepatocytes and maturation is insufficient, and a hepatocyte having high maturity has not been obtained as yet.

At present, the RNA interference (RNAi) technique is frequently utilized for the research of life sciences, and its usefulness has widely been confirmed. RNAi refers to a phenomenon wherein mRNA is degraded by double stranded RNA in a sequence-specific manner, thereby suppressing the gene expression. Ever since a report in 2001 teaching that low molecule double stranded RNA with 21 bases can mediate RNAi in the cell of a mammal (non-patent document 3), siRNA (small interference RNA) has been frequently used as a method of suppressing expression of a target gene. siRNA is expected to be applicable to pharmaceutical products, and treatment of various refractory diseases including cancer.

microRNA (miRNA) is a short chain RNA molecule composed of about 22 base pairs, which was found in nematode in 1993, which is known to bind to a complementary sequence in the 3' untranslated region of messenger RNA of the target gene to suppress the translation thereof and regulate various biological phenomena. It has been reported that human contains 1921 kinds and mouse contains 1157 kinds of microRNAs (http://www.mirbase.org/). Interestingly, plural target genes exist for one microRNA, and they are often involved in the same physiological function and the same signal transduction pathway. Therefore, variation in the expression of only one kind of microRNA can cause a dynamic change in the physiological action.

It has been suggested heretofore that microRNA plays an important role in the differentiation of hepatocytes. Non-patent document 4 discloses that stable overexpression of miR-122 in HepG2 (cell line derived from human liver cancer but used due to similarity to immature/undifferentiated hepatoblast) suppresses cell proliferation, and a further increase in the copy number of miR-122 changes the cell morphology as well as suppresses cell proliferation, and increases expression of cytochrome P450 family genes that are expressed in mature hepatocytes, thus suggesting that miR-122 plays an important role in the differentiation of hepatocytes in the development of the liver. Non-patent document 5 discloses that transfection of bipotent murine embryonic liver (BMEL) cells with miR-122 mimics increases expression of hepatocyte-specific gene, and differentiation of hepatocytes is promoted in transgenic mouse with increased miR-122 expression, and shows that miR-122 positively regulates differentiation into hepatocytes. Non-patent document 6 discloses that transfection of resident liver stem cells (RLSC-derived hepatocytes) differentiated into hepatocytes with an inhibitor of miRs-200a, b or c results in a mesenchymal-like morphology, increased expression of mesenchymal marker and an increase in the cell expressing stem cell markers, thus suggesting involvement of these microRNAs in the differentiation into hepatocytes. Non-patent document 7 discloses the analysis results of miRNA expression patterns of hepatocytes differentiated from human embryonic stem cells (hESCs) via definitive endoderm (DE) by a known method, which suggest that various miRNAs function in a cell line specific manner in the process of differentiation from hESCs into hepatocytes.

While involvement of miR-148a in the growth of some types of cancer is suggested, whether it is involved in the differentiation into hepatocytes is not known at all.

It is heretofore known that the mRNA level of CYP gene increases by treating HepG2 cells for 2 days with 5-aza-2-deoxycytidine that inhibits DNA methylation caused by Dnmt-1 (non-patent document 8). However, an increase in the CYP expression at the protein level has not been reported. On the other hand, it is known by study using HepG2 cells that miR-148a suppresses translation of PXR that promotes gene transcription of CYP3A4 (non-patent document 9). Furthermore, it has been reported that a substance inhibiting DNA methylation caused by Dnmt-1 increases expression of miR-148a (non-patent document 10). Since the expression of miR-148a increases by the inhibition of DNA methylation caused by Dnmt-1, expression of PXR is suppressed, and an increase in the mRNA expression amount of CYP gene may not be reflected on the increase at the protein level (non-patent document 11).

In addition, 5-aza-2-deoxycytidine that inhibits DNA methylation caused by Dnmt-1 has been reported to show high cytotoxicity and induce apoptosis (non-patent document 12).

### [Document List]

### [non-patent documents]

non-patent document 1: Molecular Therapy, Vol.20(1), p.127-137 (2012).
non-patent document 2: Biochemical and Biophysical Research Communications, Vol.328(1), p.258-264(2005).
non-patent document 3: Nature, Vol.411, p.494-8 (2001).
non-patent document 4: HEPATOLOGY, Vol.52(4), p.1431-1442 (2010).
non-patent document 5: GASTROENTEROLOGY, Vol.142(1), p.119-129 (2012).
non-patent document 6: Cell Death Differ, Vol.19(6), p.937-946 (2012) (doi: 10.1038/cdd.2011.175. Epub 2011 Dec 2, 1-10).
non-patent document 7: Hepatology Research, Vol.41, p.170-183 (2011).
non-patent document 8: BMC Genomics. 2006,7;181.
non-patent document 9: Journal of Biological Chemistry, Vol. 283, p.9674-9680 (2008).
non-patent document 10: Proceedings of the National Academy of Sciences of the United States of America, Vol. 105, p.13556-13561 (2008).
non-patent document 11: Epigenetic regulation of pharmacokinetics-associated genes expression by DNA methylation Atsushi Miyajima, Graduate School of Pharmaceutical Sciences, Chiba University, i-yaku-haku "KOU" No. 93.
non-patent document 12: Cancer Res, Vol.66(5), p.2794-2800(2006).

### Summary of the Invention

### Problems to be Solved by the Invention

It is known that hepatocytes produced from stem cells by differentiation induction by a conventional method have problems in that the drug-metabolizing enzyme activity (CYP activity) and the like are low and maturation is insufficient, as compared to human primary cultured hepatocytes. An object of the present invention is to provide a method of producing hepatocytes (preferably hepatocyte with high maturity) in a short period by promoting differentiation from hepatic progenitor cells into hepatocytes.

### Means of Solving the Problems

The present inventors have conducted intensive studies in an attempt to achieve the above-mentioned object and found that miR-148a is highly expressed in adult mouse hepatic tissues as compared to mouse embryonic hepatic tissue, newborn mouse hepatic tissue and mouse liver cancer-derived cell line (Hepa1-6), and that miR-148a is highly expressed in human normal hepatic tissue as compared to human liver cancer-derived cell lines (Huh-7, HepG2 and Hep3B). miR-148a precursor was introduced into hepatic progenitor cells and they were induced to differentiate into hepatocytes by culturing in a differentiation induction medium. As a result, the expression levels of albumin, glucose-6-phosphatase (G6pc), tyrosine aminotransferase (Tat), cytochrome P450 (Cyp) and miR-122, which are indices of liver function expression, remarkably increased in a short period as compared to the absence of introduction of miR-148a precursor. Furthermore, they have found that the expression level of Dnmt-1, which is a DNA methyltransferase, decreases after differentiation induction from hepatic progenitor cells into hepatocytes, namely, in inverse correlation with miR-148a expression level, and the expression level of Dnmt-1 further decreases in hepatic progenitor cells introduced with miR-148a precursor. These findings show that the expression of Dnmt-1 can be suppressed by utilizing miR-148a, whereby hepatocyte with high maturity and showing increased expression of hepatocyte marker can be obtained in a short period. In fact, when hepatic progenitor cell introduced with siRNA (siDnmt-1) that suppresses expression of Dnmt-1 was induced to differentiate into hepatocyte, the expression levels of albumin, G6pc, Tat and Cyp remarkably increased as compared to when control siRNA that does not suppress expression of Dnmt-1 was introduced. From the above findings, the present inventors have found that differentiation into hepatocyte can be promoted by suppressing expression of Dnmt-1 and conducted further studies, which resulted in the completion of the present invention.

Accordingly, the present invention relates to the following.
[1] A promoter of differentiation from hepatic progenitor cell into hepatocyte, which comprises a substance that suppresses expression of Dnmt-1 or a substance that inhibits function of Dnmt-1 as an active ingredient.
[2] The differentiation promoter of [1], wherein the substance that suppresses expression of Dnmt-1 is a nucleic acid that suppresses expression of Dnmt-1 or a precursor thereof.
[3] The differentiation promoter of [2], wherein the nucleic acid that suppresses expression of Dnmt-1 or precursor thereof is miRNA for mRNA of Dnmt-1 gene or a precursor thereof, or siRNA for mRNA of Dnmt-1 gene or a precursor thereof.
[4] The differentiation promoter of [3], wherein the nucleic acid that suppresses expression of Dnmt-1 or precursor thereof is miR-148a or a precursor thereof.
[5] The differentiation promoter of [1], wherein the substance that inhibits function of Dnmt-1 is a substance that inhibits DNA methylation caused by Dnmt-1.
[6] The differentiation promoter of [1], wherein the substance that inhibits DNA methylation caused by Dnmt-1 is a cytidine analog or a salt, solvate, hydrate, precursor or derivative thereof.
[7] The differentiation promoter of [5], wherein the substance that inhibits DNA methylation caused by Dnmt-1 masks a Dnmt-1 target sequence.
[8] The differentiation promoter of [5], wherein the substance that inhibits DNA methylation caused by Dnmt-1 blocks an active site of Dnmt-1.
[9] The differentiation promoter of any of [1] - [8], wherein the hepatic progenitor cell is a primary cultured cell or a cell induced to differentiate from a stem cell.
[10] The differentiation promoter of [9], wherein the hepatic progenitor cell is a primary cultured cell.
[11] The differentiation promoter of [9], wherein the hepatic progenitor cell is a cell induced to differentiate from an embryonic stem cell, an artificial pluripotent stem cell or a mesenchymal stem cell.
[12] A method of producing a hepatocyte from a hepatic progenitor cell, comprising the following steps:
   (1) a step of suppressing expression of Dnmt-1 or inhibiting function of Dnmt-1 in a hepatic progenitor cell by contacting the hepatic progenitor cell with the substance that suppresses expression of Dnmt-1 or substance that inhibits function of Dnmt-1 of any of [1] - [9]; and
   (2) a step of inducing differentiation into a hepatocyte by culturing the hepatic progenitor cell obtained in the aforementioned step (1), wherein expression of Dnmt-1 is suppressed or function of Dnmt-1 is inhibited.
[13] The method of [12], wherein the hepatic progenitor cell is a primary cultured cell a cell induced to differentiate from or a stem cell.
[14] The method of [13], wherein the hepatic progenitor cell is a primary cultured cell.
[15] The method of [13], wherein the hepatic progenitor cell is a cell induced to differentiate from an embryonic stem cell, an artificial pluripotent stem cell or a mesenchymal stem cell.
[16] A hepatocyte produced by the method of any of [12] - [15].
[17] A method of evaluating metabolism of a test compound, comprising the steps of the following (a) and (b):
   (a) a step of contacting the hepatocyte of [16] with a test compound; and
   (b) a step of measuring the metabolism of the test compound contacted with the hepatocyte in the aforementioned step (a).
[18] A method of evaluating hepatotoxicity of a test compound, comprising the steps of the following (a) and (b):
   (a) a step of contacting the hepatocyte of [16] with a test compound; and
   (b) a step of measuring the level of disorder of the hepatocyte contacted with the test compound in the aforementioned step (a).
[19] A promoter of differentiation from hepatoblastoma into hepatocyte, which comprises a substance that suppresses expression of Dnmt-1 or a substance that inhibits function of Dnmt-1 as an active ingredient.
[20] A method of producing a hepatocyte from a hepatoblastoma, comprising the following steps:
   (1) a step of suppressing expression of Dnmt-1 or inhibiting function of Dnmt-1 in a hepatoblastoma by contacting the hepatoblastoma with the substance that suppresses expression of Dnmt-1 or substance that inhibits function of Dnmt-1 of any of [1] - [9]; and
   (2) a step of inducing differentiation into a hepatocyte by culturing the hepatoblastoma obtained in the aforementioned step (1), wherein expression of Dnmt-1 is suppressed or function of Dnmt-1 is inhibited for not less than 7 days.

### Effect of the Invention

According to the present invention, differentiation from a hepatic progenitor cell or hepatoblastoma into a hepatocyte can be promoted. As a result, a hepatocyte having higher maturity than hepatocytes produced by inducing differentiation from a stem cell by a conventionally-known method can be provided in a short period.

### Brief Description of the Drawings

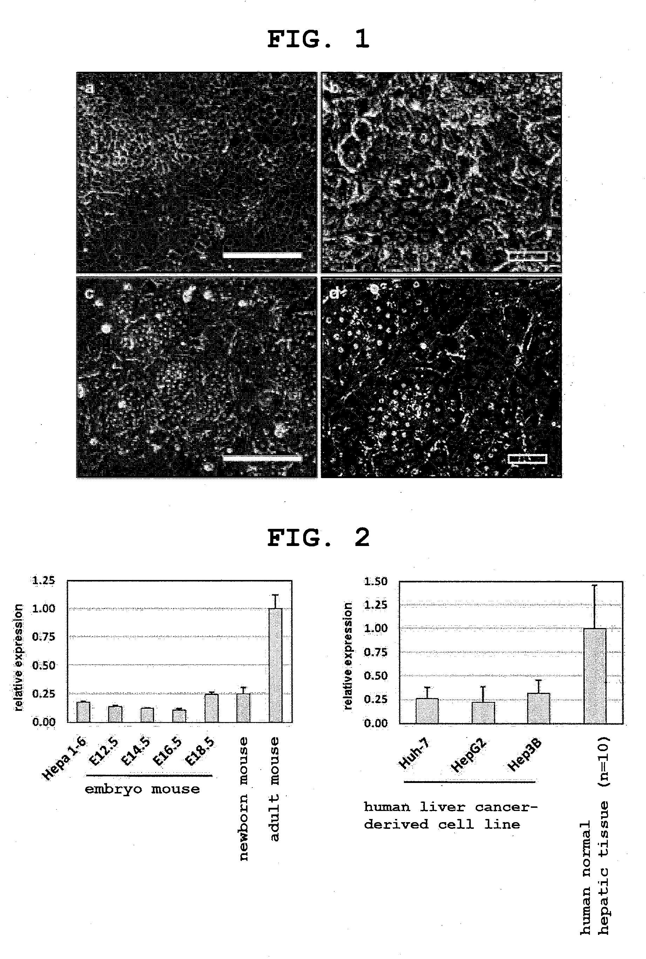

Fig. 1 shows changes in the morphology of hepatic progenitor cells after differentiation induction. The cultured cells reached confluent 3 days after culture and the cell morphology was quadrate (a). Five days after culture, the cell morphology became oval (b). Twelve days after culture, the cell morphology became that of matured hepatocyte (c and d). Scale bar: 200 µm (a, c), 50 µm (b, d).
Fig. 2 shows the expression levels of miR-148a in hepatic tissues or liver cancer-derived cell lines of human (right panel) and mouse (left panel). mean±SEM (n=10).

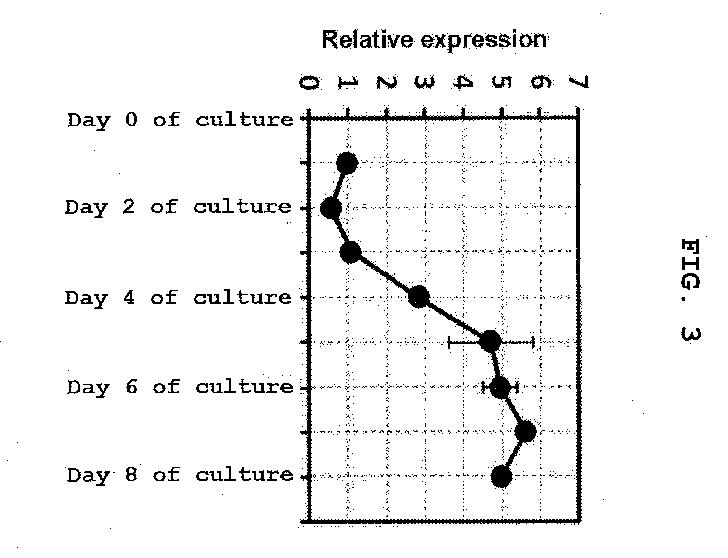

Fig. 3 shows changes in the expression level of miR-148a after differentiation induction in hepatic progenitor cells. mean±SEM (n=3).

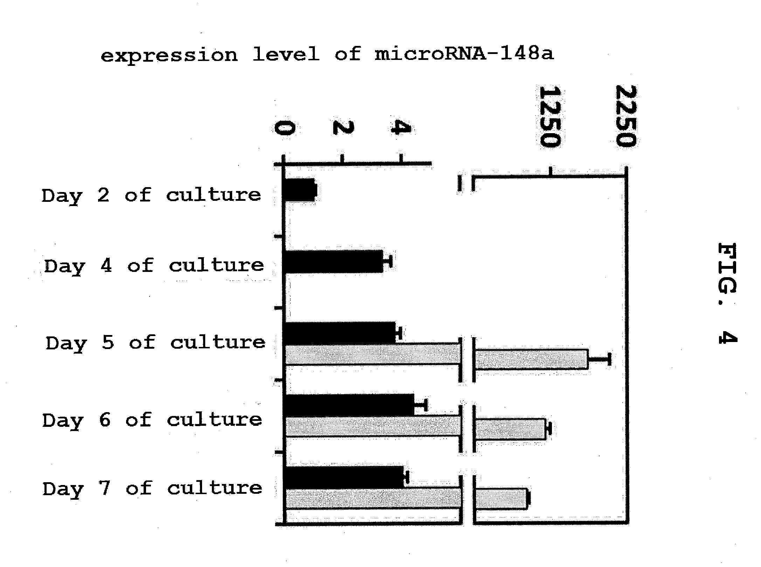

Fig. 4 shows changes in the expression level of miR-148a after differentiation induction in hepatic progenitor cells introduced with miR-148a precursor. The black bar shows the expression level of hepatic progenitor cells not introduced with miR-148a precursor, and the gray bar shows the expression level of hepatic progenitor cells introduced with miR-148a precursor. mean±SEM (n=3).

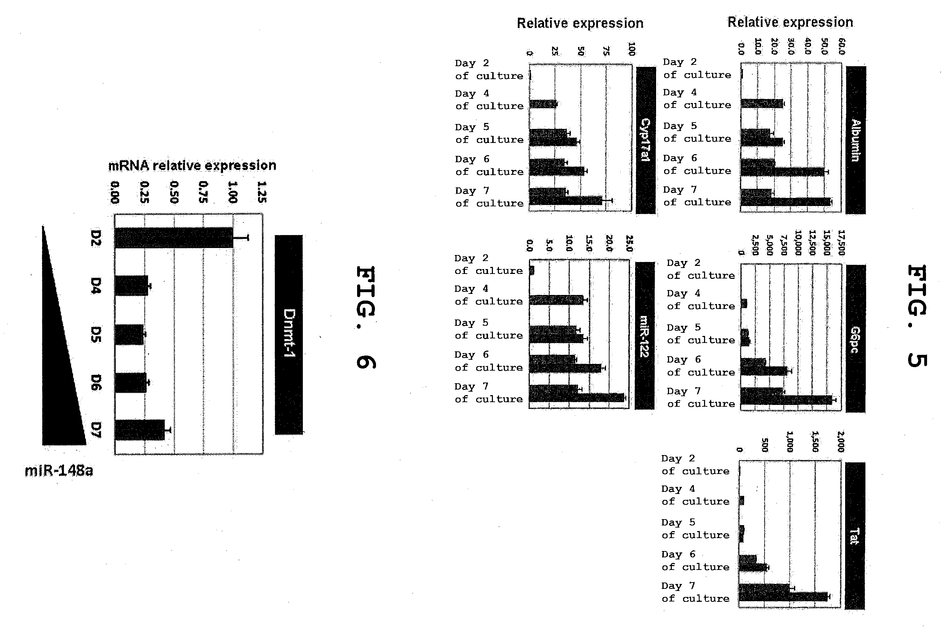

Fig. 5 shows changes in the expression level of albumin, G6pc, Tat, Cyp17a1 and miR-122 after differentiation induction in hepatic progenitor cells introduced with miR-148a precursor. The bar on the left side shows the expression level in hepatic progenitor cells not introduced with miR-148a, and the bar on the right side shows the expression level in hepatic progenitor cells introduced with miR-148a precursor. mean±SEM (n=3).
Fig. 6 shows changes in the expression level of Dnmt-1 after differentiation induction in hepatic progenitor cells. mean±SEM (n=3).

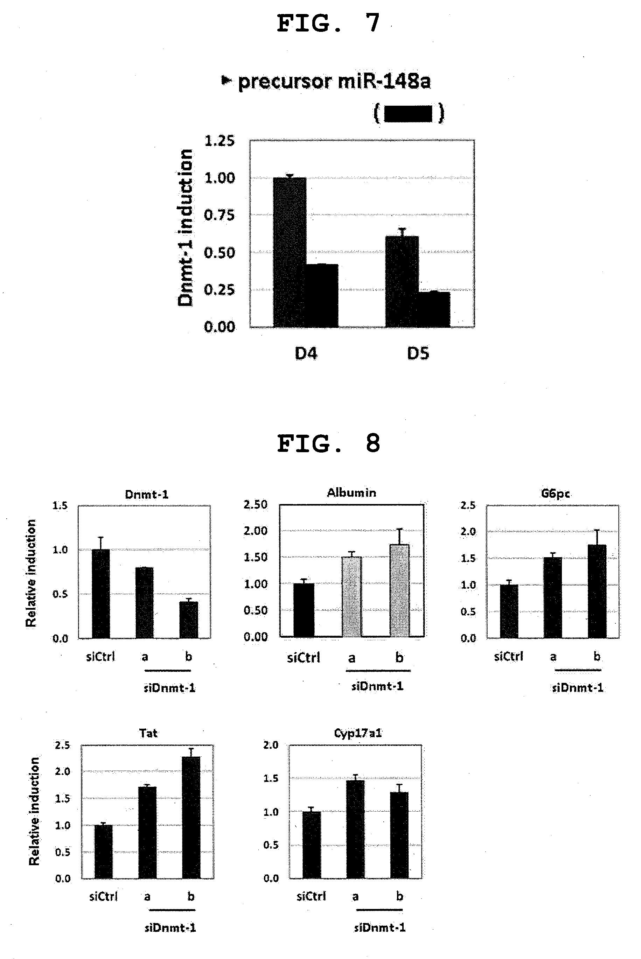

Fig. 7 shows the expression level of Dnmt-1 on day 4 (D4) and day 5 (D5) after the start of culture in hepatic progenitor cells introduced with miR-148a precursor. The bar on the left side shows the expression level in hepatic progenitor cells not introduced with miR-148a, and the bar on the right side shows the expression level in hepatic progenitor cells introduced with miR-148a precursor. mean±SEM (n=3).
Fig. 8 shows changes in the expression level of Dnmt-1, albumin, G6pc, Tat and Cyp17a1 on day 6 after the start of culture in hepatic progenitor cell introduced with siRNA (siDnmt-1) that suppresses expression of Dnmt-1. "siCtrl" shows the expression level in hepatic progenitor cell introduced with siRNA (siCtrl) that does not suppress expression of Dnmt-1, and "siDnmt-1" shows the expression level in hepatic progenitor cell introduced with siDnmt-1 (a and b show two kinds of siRNAs (siDnmt-1) with Dnmt-1 as target gene, whose base sequences are different). mean±SEM (n=3).

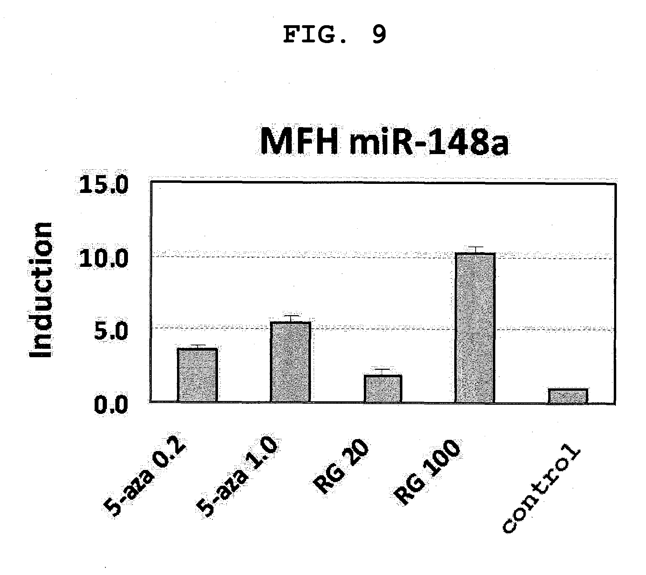

Fig. 9 shows changes in the expression level of miR-148a after differentiation induction in hepatic progenitor cells cultured in a medium added with substance that inhibits DNA methylation caused by Dnmt-1. mean±SEM (n=3).

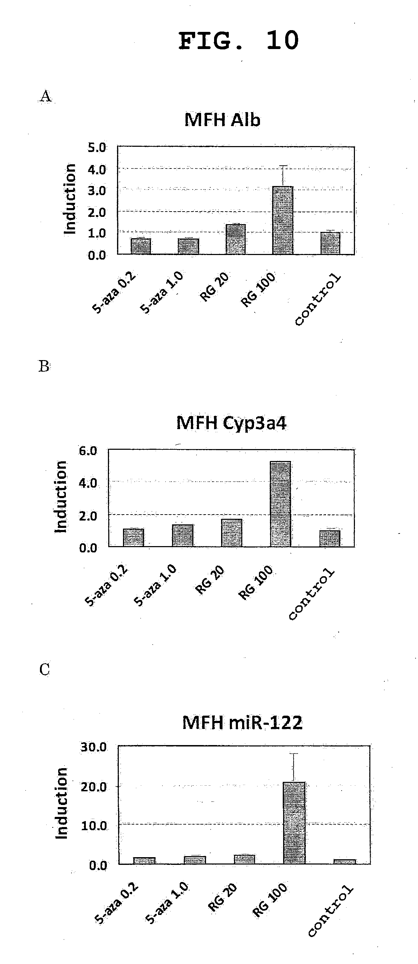

Fig. 10 shows changes in the expression level of hepatocyte marker after differentiation induction in hepatic progenitor cells cultured in a medium added with substance that inhibits DNA methylation caused by Dnmt-1. mean±SEM (n=3).

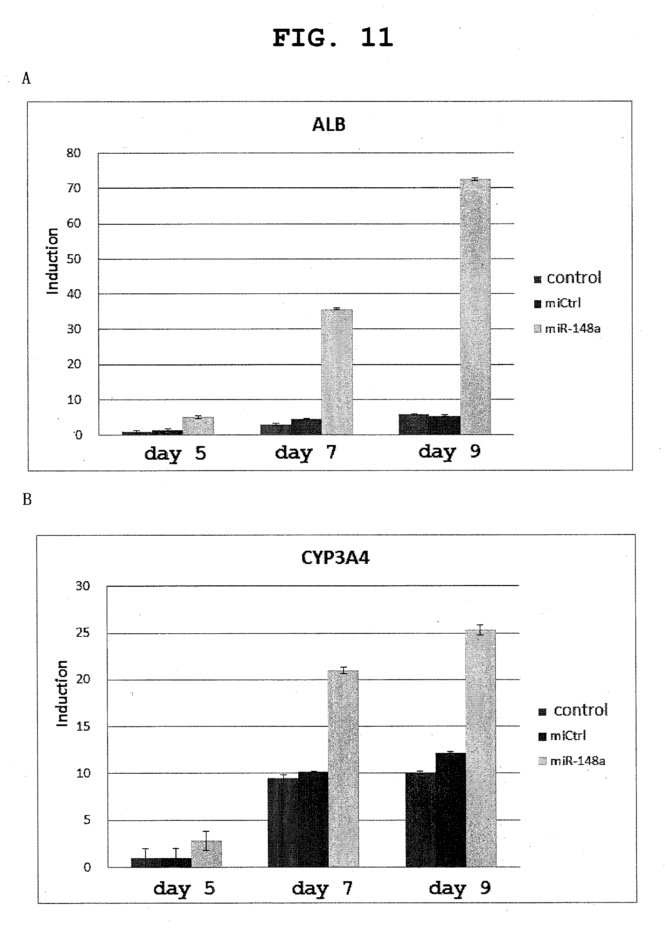

Fig. 11 shows the expression levels of (A) albumin (ALB) and (B) Cyp3a4 in human hepatic progenitor cell cultured in a medium alone (control), human hepatic progenitor cell introduced with miR-148a precursor (miR-148a), and human hepatic progenitor cell introduced with miRNA without function (miCtrl) after differentiation induction. The bar on the left side shows the expression level in cells introduced with control, the middle bar shows the expression level in cells introduced with miCtrl, and the bar on the right side shows the expression level in cells introduced with miR-148a.

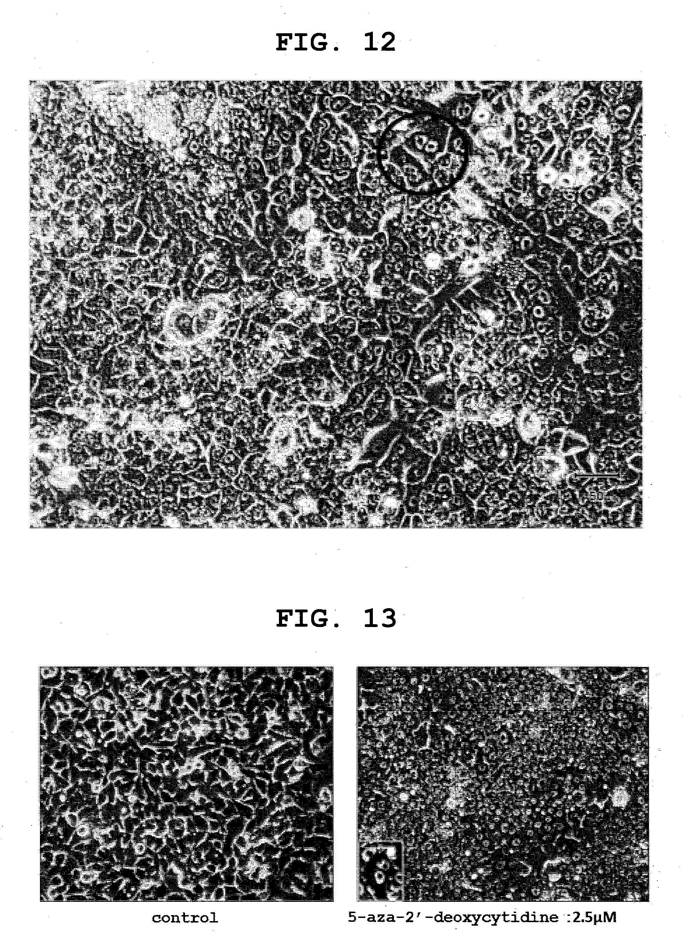

Fig. 12 shows observation, under an optical microscope, of human hepatic progenitor cells introduced with miR-148a precursor. A cell having a morphology with multiple nuclei and a clear cell boundary, which are specific to mature hepatocytes, is shown enclosed with a circle in the figure. scale bar: 50 µm.
Fig. 13 shows the cell morphology of HepG2 cells after treating with 5-aza-2'-deoxycytidine (2.5 µM) which is a substance that inhibits DNA methylation caused by Dnmt-1. Cells cultured under the same conditions except the treatment with 5-aza-2'-deoxycytidine were used as a control.

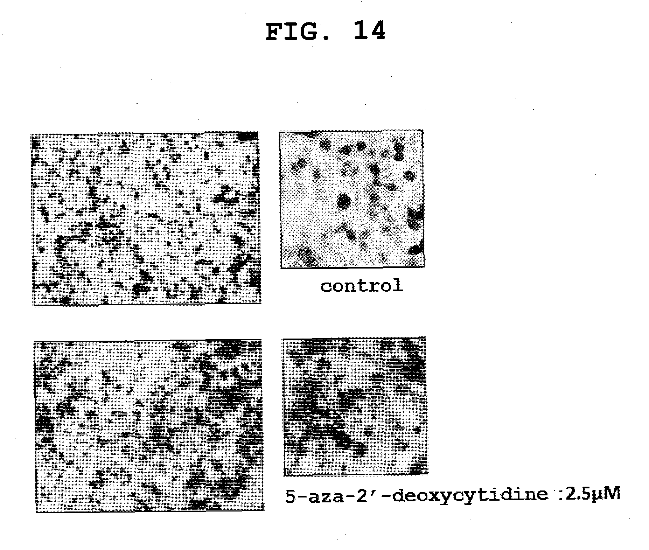

Fig. 14 shows the results of PAS staining of HepG2 cells after treatment with 5-aza-2'-deoxycytidine (2.5 µM). Cells cultured under the same conditions except the treatment with 5-aza-2'-deoxycytidine were used as a control.

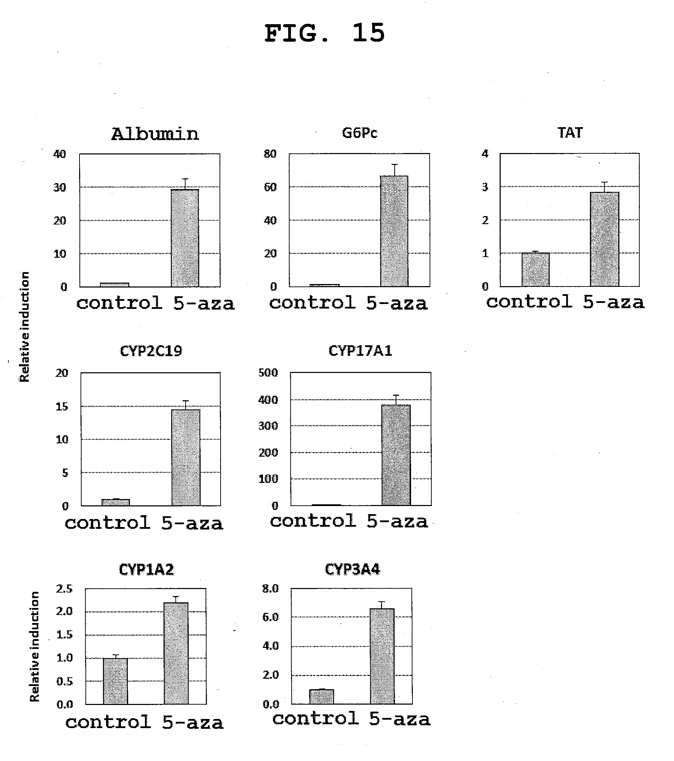

Fig. 15 shows changes in the expression level of hepatocyte marker in HepG2 cells after treating with 5-aza-2'-deoxycytidine (5-aza) (2.5 µM). Cells cultured under the same conditions except the treatment with 5-aza-2'-deoxycytidine were used as a control.

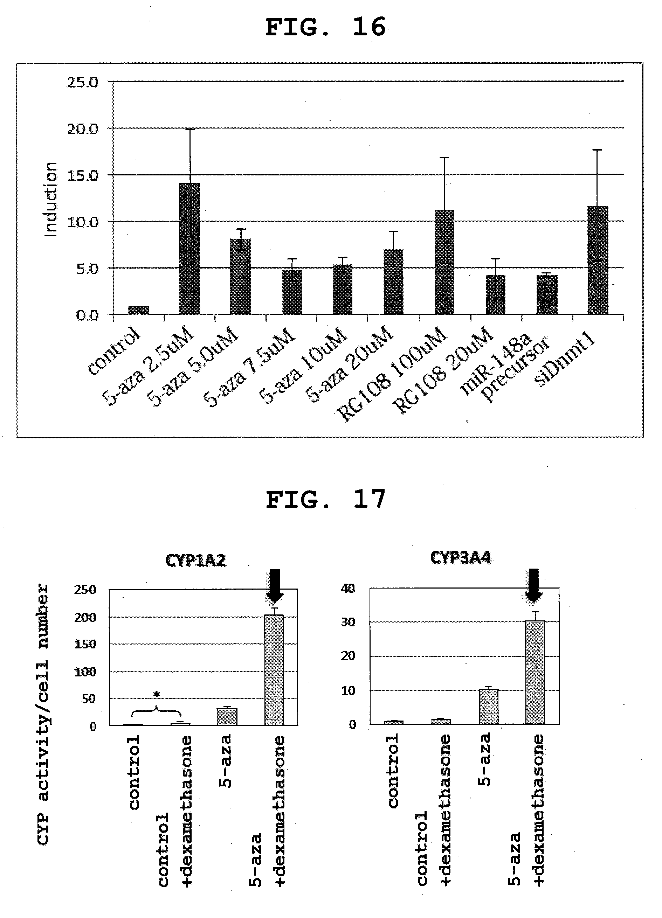

Fig. 16 shows the measured activity of CYP enzyme in HepG2 cells after treatment with a substance that inhibits DNA methylation caused by Dnmt-1 (5-aza-2'-deoxycytidine (5-aza) or RG108), or an inhibitory nucleic acid for Dnmt-1 (miR-148a or siDnmt1). Cells cultured under the same conditions except the treatment with the inhibitory substance and the inhibitory nucleic acid were used as a control.
Fig. 17 shows the measured activity of CYP enzyme (CYP1A2 or CYP3A4) in HepG2 cells after treatment with 5-aza-2'-deoxycytidine (5-aza) (2.5 µM) followed by enzyme induction with dexamethasone (25 µM). Cells cultured under the same conditions except the treatment with 5-aza-2'-deoxycytidine were used as a control.

### Description of Embodiments

### 1. Differentiation promoter from hepatic progenitor cell or

### hepatoblastoma into hepatocyte

The present invention provides a promoter of differentiation from a hepatic progenitor cell into a hepatocyte, which contains, as an active ingredient, a substance that suppresses expression of Dnmt-1 or a substance that inhibits function of Dnmt-1. In a further aspect, the present invention provides a promoter of differentiation from a hepatoblastoma into a hepatocyte, which contains, as an active ingredient, a substance that suppresses expression of Dnmt-1 or a substance that inhibits function of Dnmt-1. The promoter of differentiation from a hepatic progenitor cell into a hepatocyte, and the promoter of differentiation from a hepatoblastoma into a hepatocyte above are collectively referred to as the promoter of the present invention. Dnmt-1 is one of the DNA methyltransferases of mammals. The present invention is based on the finding that the expression of hepatocyte marker is promoted in differentiation induction from a hepatic progenitor cell or hepatoblastoma into a hepatocyte by suppressing the expression of Dnmt-1 or inhibiting the function of Dnmt-1.

The promoter of the present invention after incorporation into a cell shows an activity to suppress expression of Dnmt-1, or inhibit the function of Dnmt-1 activity. The activity to suppress expression of Dnmt-1 can be examined by using a transformant transfected with Dnmt-1 gene, Dnmt-1 gene expression system in vivo or in vitro, or Dnmt-1 translation system Dnmt-1 in vivo or in vitro. In addition, the activity to inhibit function of Dnmt-1 can be examined by using in vivo or in vitro DNA methyltransferase activity measurement system of Dnmt-1 known per se. For example, the promoter of the present invention can be judged to have a Dnmt-1 function inhibitory activity when a cell that expresses Dnmt-1 is cultured in the presence of the promoter of the present invention, the methylation states of the parent cell DNA and the daughter cell DNA are examined, and the methylation state of the parent cell DNA is not transmitted to the daughter cell DNA.

The promoter of the present invention is introduced into a hepatic progenitor cell or hepatoblastoma and promotes differentiation into a hepatocyte. In the present invention, the "hepatocyte" is a cell that expresses at least one kind of hepatocyte markers (e.g., albumin, glucose-6-phosphatase (G6pc), tyrosine aminotransferase (Tat), cytochrome P450 (Cyp), miR-122 etc.) (preferably, 4 kinds of albumin, G6pc, Tat and Cyp, more preferably 5 kinds of albumin, G6pc, Tat, Cyp and miR-122). To "promote differentiation into hepatocyte" means to increase the expression level of at least one kind of hepatocyte markers (e.g., albumin, glucose-6-phosphatase (G6pc), tyrosine aminotransferase (Tat), cytochrome P450 (Cyp), miR-122 etc.) (preferably, 4 kinds of albumin, G6pc, Tat and Cyp, more preferably, 5 kinds of albumin, G6pc, Tat, Cyp and miR-122) after a lapse of a given number of days (e.g., 1 day, 2 days, 3 days, 4 days, 5 days, 6 days, 7 days etc.) from the start of differentiation induction, as compared to differentiation induction of hepatic progenitor cell or hepatoblastoma not introduced with the promoter of the present invention. In this case, differentiation induction is performed by culturing the cells in William's E medium (Invitrogen Corporation) added with 2 mM L-glutamine solution (Invitrogen Corporation), 50 U/mL penicillin-50 µg/mL streptomycin solution (Invitrogen Corporation), 10% fetal bovine serum (Thermo Fisher Scientific K.K.), 5 µg/mL insulin (Sigma-Aldrich Corporation), 25 ng/mL epidermal growth factor (EGF) (Sigma-Aldrich Corporation), 25 ng/mL hepatocyte growth factor (HGF) (Peprotech), 12.5 ng/mL oncostatin M (Sigma-Aldrich Corporation), 5×10⁻⁷M hydrocortisone 21-hemisuccinate sodium salt (Sigma-Aldrich Corporation), 10⁻⁷ M dexamethasone (Sigma-Aldrich Corporation) for 48 hr, and thereafter culturing the cells in a medium free of epidermal growth factor (EGF) and fetal bovine serum. Alternatively, in a futher aspect, differentiation induction of hepatoblastoma can be performed by, for example, continuous cultivation in a DMEM medium (Invitrogen Corporation) added with 2 mM L-glutamine solution (Invitrogen Corporation), 50 U/mL penicillin-50 µg/mL streptomycin solution (Invitrogen Corporation), 10% fetal bovine serum (Thermo Fisher Scientific K.K.). The expression level of a hepatocyte marker can be measured by a method known per se. For example, total RNA is extracted from a cell, and the mRNA level or miRNA level of the hepatocyte marker may be quantified by a known method such as real-time PCR method and the like.

In the present invention, the hepatic progenitor cell means a cell having proliferative capacity and an ability to differentiate into two directions of hepatocyte and bile duct epithelial cell, for which E-cadherin has been identified as a surface antigen marker. The hepatic progenitor cell also includes hepatic stem cells and hepatoblasts.

The hepatic progenitor cell used in the present invention is not particularly limited as long as it has the above-mentioned properties, and is preferably a non-tumor cell.

The hepatic progenitor cell used in the present invention is generally a cell of a mammal (e.g., rat, mouse, guinea pig, rabbit, sheep, horse, swine, bovine, monkey, human, preferably human).

The promoter of the present invention can promote differentiation into hepatocyte as long as the cell is a hepatic progenitor cell, irrespective of the method of obtaining same. Hepatic progenitor cell can be obtain by a method known per se, and examples thereof include, but are not limited to, cells induced to differentiate from primary cultured cell obtained from a living organism, stem cell (embryonic stem cell (ES cell), induced pluripotent stem cell (iPS cell), mesenchymal stem cell etc.) and the like.

In one embodiment, hepatic progenitor cell is a primary cultured cell. For example, hepatic progenitor cell can be isolated by sorting out E-cadherin positive cells from the hepatocyte group contained in the liver (preferably, viviparous stage liver) isolated from a living organism. More specifically, hepatic progenitor cell can be isolated by the following steps which are not limitative: collecting hepatic tissues from embryo, cutting them into small pieces with surgical scissors etc., and treating them with an enzyme solution containing protease (e.g., liberase (manufactured by Roche Applied Science) etc.). The dispersed cells are washed with a medium (e.g., basal medium for hepatocyte) to give hepatic parenchymal cell. The obtained hepatic parenchymal cells are contacted with a biotin-labeled anti-E-cadherin antibody (e.g., manufactured by eBioscience), and the cells labeled with the biotin-labeled antibody are recovered using EasySep Mouse Biotin Positive Selection Kit (manufactured by STEMCELL Technologies) and the like. The recovered cells are hepatic progenitor cells.

In another embodiment, the hepatic progenitor cell is a cell induced to differentiate from a stem cell. Stem cell includes embryonic stem cells (ES cell), induced pluripotent stem cell (iPS cell) and mesenchymal stem cell.

Examples of the ES cell include an embryonic stem cell of a mammal and the like, which is established by cultivating early embryo before implantation, an embryonic stem cell established by cultivating an early embryo prepared by nuclear transplantation of the nucleus of a somatic cell, and an embryonic stem cell obtained by alteration of its gene(s) on chromosome(s) by a known genetic engineering method.

To be specific, examples of the embryonic stem cell include embryonic stem cells established from an inner cell mass, single blastomere and the like constituting an early embryo, EG cell established from a primordial germ cell, a cell isolated from a cell population (e.g., primitive ectoderm) having multipotency of an early embryo before implantation, a cell obtained by cultivating said cell and the like. Also, these embryonic stem cells may be those distinguishable to have reached the corresponding differentiation stage by using expression of a marker gene as an index, by inflame knock-in of the marker gene (e.g., fluorescence protein such as GFP and the like) into a gene encoding the differentiation marker by a known method.

Embryonic stem cells are available from predetermined institutions, and commercially available products can also be purchased. For example, KhES-1, KhES-2 and KhES-3, which are human embryonic stem cells, are available from Institute for Frontier Medical Sciences, Kyoto University.

Fused ES cells obtained by cell fusion of ES cell and somatic cell are also included in the embryonic stem cells to be used for the method of the present invention.

Induced pluripotent stem cell (iPS cell) is obtained by reprogramming somatic cell (e.g., fibroblast, skin cell etc.) by introduction of a reprogramming factor. Induced pluripotent stem cell was found for the first time by a method including introducing reprogramming factors consisting of Oct3/4, Sox2, Klf4 and c-Myc into a somatic cell (e.g., fibroblast, skin cell etc.) (Cell, 126: p. 663-676, 2006). Thereafter, many researchers are working on various improvements in the combinations of reprogramming factors and introduction method of the factors, and various production methods of induced pluripotent stem cells have been reported. The induced pluripotent stem cell in the present invention also includes cells produced by such methods.

Mesenchymal stem cell is a somatic stem cell derived from mesenchyme, and has differentiation potency into a cell belonging to mesenchyme. It is also shown to have plasticity enabling differentiation into non-mesodermal tissues such as ectoderm-derived cells, endoderm-derived cells and the like. Mesenchymal stem cell includes, for example, bone marrow mesenchymal cell and cord blood-derived stem cell. While mesenchymal stem cell is considered to exist in any mesenchymal tissues, bone marrow mesenchymal stem cell can be easily collected by bone marrow puncture from among the mesenchymal tissues, and culture techniques have been established. Bone marrow mesenchymal stem cell is included in bone marrow interstitial cells, and bone marrow interstitial cell is one kind of cell that supports hematopoietic cell to be the main cell in the bone marrow. On the other hand, cord blood is the blood contained in the umbilical cord as an embryo side tissue connecting embryo and mother. While it is known that a large amount of cord blood-derived stem cells (hematopoietic stem cells) is contained in the cord blood, it has also been clarified that mesenchymal stem cells (cord blood-derived mesenchymal stem cells), which are somatic stem cells other than hematopoietic stem cell, are also present.

Induction of differentiation from stem cell into hepatic progenitor cell can be performed, for example, by culturing the cell in a culture medium added with activin A, and culturing the cell in a culture medium added with bone morphogenic protein (BMP) and fibroblast growth factor (FGF), and the like.

Hepatoblastoma is a malignant tumor developed from immature hepatoblast. Hepatoblastoma may be established, and examples of the established cell line include HepG2.

Dnmt-1 is a molecule known already, and is known to have a DNA methyltransferase activity. Conventionally, the correlation between the Dnmt-1 expression level in liver cancer cells and the malignancy level of liver cancer has been suggested (International Journal of Cancer, Volume 105, Issue 4, pages 527-532, 1 July 2003); however, the correlation between Dnmt-1 and hepatocyte differentiation is not known at all.

Amino acid sequence of Dnmt-1 and base sequence encoding same are published in several kinds of animals. For example, a base sequence and an amino acid sequence of human Dnmt-1 have been published as NCBI accession No. NM_001130823.1 (renewed on June 17, 2012) (SEQ ID NO: 1) and NP_001124295.1 (renewed on June 17, 2012) (SEQ ID NO: 2), and the base sequence and amino acid sequence of mouse Dnmt-1 have been published as NCBI accession No. NM_010066.4 (renewed on May 5, 2012) (SEQ ID NO: 3) and NP_034196.5 (renewed on May 5, 2012) (SEQ ID NO: 4), respectively. Sequence information of Dnmt-1 ortholog of other mammal and naturally-occurring variants can be obtained from public databases (e.g., HomoloGene (http://www.ncbi.nlm.nih.gov/HomoloGene/) and UniProtKB/Swiss-Protdatabase etc.) well known in the pertinent technical field.

The function of Dnmt-1 is known to be introduction of methylation state of the original DNA (Parent Strand) into a new DNA (Daughter strand) by DNA methyltransferase activity in the DNA replication process.

In the present invention, "a substance that suppresses expression of Dnmt-1" may act in any stage such as transcription level of Dnmt-1 gene encoding Dnmt-1, post-transcription regulation level, translation level into Dnmt-1, post-translational modification level and the like. Therefore, Examples of the substance that suppresses expression of Dnmt-1 include a substance that inhibits transcription of Dnmt-1 gene (e.g., antigen), a substance that inhibits processing of initial transcription product to mRNA, a substance that inhibits transportation of mRNA to the cytoplasm, a substance that inhibits translation of mRNA into Dnmt-1 (e.g., antisense nucleic acid, miRNA) or degrades mRNA (e.g., siRNA, ribozyme, miRNA), a substance that inhibits post-translational modification of initial translation product and the like. While any substance that acts on any stage can be preferably used, a substance that complementarily binds to mRNA to inhibit translation into Dnmt-1 or degrade mRNA is preferable.

Preferable examples of the substance that specifically inhibits translation of mRNA of Dnmt-1 gene into Dnmt-1 (or degrades mRNA) include a nucleic acid containing a base sequence complementary or substantially complementary to the base sequence of the mRNA, or a part thereof.

In the present invention, nucleic acid is RNA, chimeric nucleic acid of RNA and DNA (hereinafter to be referred to as chimeric nucleic acid) or hybrid nucleic acid. As used herein, chimeric nucleic acid means that a single stranded or double stranded nucleic acid contains RNA and DNA in a single nucleic acid, and hybrid nucleic acid refers to a double stranded nucleic acid having RNA or chimeric nucleic acid for one chain and DNA or chimeric nucleic acid for the other chain.

In the present invention, nucleic acid is single stranded or double stranded. Embodiments of double strand include double stranded RNA, double stranded chimeric nucleic acid, RNA/DNA hybrid, RNA/chimeric nucleic acid hybrid, chimeric nucleic acid/chimeric nucleic acid hybrid, and chimeric nucleic acid/DNA hybrid. In the present invention, nucleic acid is preferably single stranded RNA, single stranded chimeric nucleic acid, double stranded RNA, double stranded chimeric nucleic acid, RNA/DNA hybrid, RNA/chimeric nucleic acid hybrid, chimeric nucleic acid/chimeric nucleic acid hybrid or chimeric nucleic acid/DNA hybrid, more preferably single stranded RNA, single stranded chimeric nucleic acid, double stranded RNA, double stranded chimeric nucleic acid, RNA/DNA hybrid, chimeric nucleic acid/chimeric nucleic acid hybrid or RNA/chimeric nucleic acid hybrid.

The "base sequence substantially complementary to the base sequence of mRNA of Dnmt-1 gene" means a base sequence having complementarity of the level enabling, under physiological conditions of mammals, binding to the target sequence of the mRNA to inhibit translation thereof (or cleave the target sequence), and is specifically, for example, a base sequence having a homology of about not less than 80%, preferably not less than about 90%, more preferably not less than about 95%, particularly preferably not less than about 97%, with a region overlapping with a base sequence completely complementary to the base sequence of the mRNA (i.e., base sequence of complementary chain of mRNA).

The "homology of base sequence" in the present invention can be calculated using homology calculation algorithm NCBI BLAST (National Center for Biotechnology Information Basic Local Alignment Search Tool) under the following conditions (expectancy =10; gap allowed; filtering=ON; match score=1; mismatch score=-3).

The "part of a base sequence complementary or substantially complementary to the base sequence of mRNA of Dnmt-1 gene" is not particularly limited as to its length and position as long as it can specifically bind to mRNA of Dnmt-1 gene, and can inhibit translation of protein from the mRNA (or degrade the mRNA).

Specific examples of the nucleic acid containing a base sequence complementary or substantially complementary to the base sequence of mRNA of Dnmt-1 gene, or a part thereof preferably include the following (a) and (b).
(a) miRNA to mRNA of Dnmt-1 gene or a precursor thereof
(b) siRNA to mRNA of Dnmt-1 gene or a precursor thereof

### (a) miRNA to mRNA of Dnmt-1 gene or a precursor thereof

In the present invention, miRNA is a 17 - 28 base length single stranded RNA, which complementarily binds to target mRNA to inhibit translation of the mRNA (or degrade the mRNA), thus regulating gene expression after transcription. miRNA to mRNA of Dnmt-1 gene may be any miRNA as long as it suppresses expression of Dnmt-1. As a mechanism of suppression of the translation of mRNA of the target gene by miRNA, it is known that mRNA having a base sequence complementary to the 2nd - 8th base sequence on the 5'-terminal side of miRNA is recognized as an miRNA target gene by miRNA (Current Biology, 15, R458-R460 (2005)). By this mechanism, expression of the mRNA is suppressed by miRNA. Accordingly, miRNA to mRNA of Dnmt-1 gene preferably has, in the 2nd - 8th base sequence on the 5'-terminal side, a base sequence complementary to a base sequence in mRNA of Dnmt-1 gene (preferably in 3'UTR of mRNA). Examples of such miRNA include miR-148a.

miR-148a means an already-known molecule typically called mature miRNA. As used herein, miR-148a includes those derived from any species and examples thereof include molecules derived from human (has-miR-148a (also called has-miR-148a-3p): base sequence shown in SEQ ID NO: 5, registered in miRBase under Accession No. MIMAT0000243), mouse (mmu-miR-148a (also called mmu-miR-148a-3p): base sequence shown inSEQ ID NO: 6, registered in miRBase under Accession No. MIMAT0000516) and the like. In the present invention, miR-148a may be derived from the same or different species from which the cell to be applied with the promoter of the present invention derives, as long as it suppresses expression of Dnmt-1 in the cell to be applied with the promoter of the present invention. In the present invention, a nucleic acid "derived from organism X" means that the base sequence of the nucleic acid contains a base sequence the same or substantially the same as the base sequence of said nucleic acid existing naturally present in the organism X. Being "substantially the same" means that the noted base sequence has an identity of not less than 70% (preferably not less than 80%, more preferably not less than 90%, further preferably not less than 98%, most preferably not less than 99%, with a base sequence of a nucleic acid naturally present in organism X, and maintains the function of the nucleic acid.

miRNA may be introduced into a cell as a mature miRNA, or introduced into a cell as a precursor thereof. A precursor of miRNA means a nucleic acid capable of producing mature miRNA in a cell, as a result of intracellular processing or cleavage of double stranded nucleic acid. Examples of the precursor include pri-miRNA, pre-miRNA and the like. pri-miRNA is a primary transcription product of miRNA gene (single stranded RNA), and generally has a length of about several hundred to several thousand bases. pre-miRNA is a single stranded RNA having a hairpin structure formed by intranuclear processing of pri-miRNA, and generally has a length of 90 - 110 bases. Since these precursors of miRNAs are naturally-occurring substances, the possibility of being eliminated when introduced in a large amount, or causing cell responses such as interferon response and the like is considered to be low. Examples of the precursor of miRNA to mRNA of Dnmt-1 gene include the precursor of has-miR-148a (base sequence shown in SEQ ID NO: 7, registered in miRBase under Accession No. MI0000253), the precursor of mmu-miR-148a (base sequence shown in SEQ ID NO: 8, registered in miRBase under Accession No. MI0000550) and the like.

In another preferable embodiment, a nucleic acid containing a nucleotide having a similar activity as that of mature miRNA (i.e. activity to suppress expression of Dnmt-1), for example, an miRNA mimic synthesized to mimic endogenous mature miRNA and the like can be used as miRNA. A commercially available one can also be utilized.

miRNA can contain various chemical modifications to improve stability (chemical and/or to enzymatic) and specific activity (affinity for RNA). For example, to prevent decomposition by hydrolase such as nuclease and the like, phosphate residue (phosphate) of each nucleotide constituting miRNA can be substituted by, for example, a chemically modified phosphate residue such as phosphorothioate (PS), methylphosphonate, phosphorodithionate and the like. In addition, the 2'-position hydroxyl group of sugar (ribose) of each nucleotide may be substituted by OR [R=CH₃(2'-O-Me), F(2'-F), CH₂CH₂OCH₃(2'-O-MOE), CH₂CH₂NHC(NH)NH₂, CH₂CONHCH₃, CH₂CH₂CN etc.]. Furthermore, the base region (pyrimidine, purine) may be chemically modified and, for example, introduction of methyl group or cationic functional group into the 5-position of pyrimidine base, substitution of the 2-position carbonyl group by thiocarbonyl and the like can be mentioned.

The conformation of the sugar region of nucleic acid is dominantly C2'-endo(S type) or C3'-endo(N type); in a single stranded nucleic acid, the two are present in equilibrium and, when a double strand is formed, it is fixed to N type. To impart strong bindability to target RNA, therefore, LNA which is a nucleic acid derivative wherein the conformation of the sugar region is fixed to N type by crosslinking 2' oxygen and 4' carbon via a methylene residue (Imanishi, T. et al., Chem. Commun., 1653-9, 2002; Jepsen, J.S. et al., Oligonucleotides, 14, 130-46, 2004) and ENA which is a nucleic acid derivative wherein the conformation of the sugar region is fixed to N type by crosslinking 2' oxygen and 4' carbon via an ethylene residue (Morita, K. et al., Nucleosides Nucleotides Nucleic Acids, 22, 1619-21, 2003) can also be used preferably.

miRNA can be prepared, for example, by obtaining the information of the base sequence of miRNA of interest from miRBase database and the like, and synthesizing, based thereon, by a commercially available DNA/RNA automatic synthesizer (Applied Biosystems, Beckman Instruments etc.). The above-mentioned miRNA containing various modifications can also be chemically synthesized by a method known per se. Commercially available miRNAs such as has-miR-148a-3p (Life Technologies Corporation) can also be utilized.

### (b) siRNA to mRNA of Dnmt-1 gene or a precursor thereof

In the present specification, double stranded RNA consisting of oligoRNA complementary to mRNA of Dnmt-1 gene and a complementary chain thereof, namely siRNA, is also defined to be encompassed in a nucleic acid containing a base sequence complementary or substantially complementary to the base sequence of mRNA of Dnmt-1 gene or a part thereof. When a short double stranded RNA is intracellularly introduced, mRNA complementary to RNA thereof is degraded, which is a phenomenon called RNA interference (RNAi) known for long in nematode, insect, plant and the like. Ever since this phenomenon was confirmed to widely occur in animal cells as well [Nature, 411(6836): 494-498 (2001)], it has been widely used as alternative technology for ribozyme.

siRNA to mRNA of Dnmt-1 gene is not particularly limited as long as it has RNAi activity for mRNA of Dnmt-1 gene, and can be designed based on the cDNA sequence information of the target gene and in accordance with, for example, the rules proposed by Elbashir et al. (Genes Dev., 15, 188-200 (2001)). Examples of the target sequence of siRNA include, but are not limited to, AA+(N)19, AA+(N)21 or NA+(N)21 (N is any base) and the like. Also, the position of the target sequence is not particularly limited. The selected candidate group of the target sequences is examined for the presence of homology with a sequence of 16-17 continuous bases in mRNA other than the target by using a homology search software such as BLAST (http://www.ncbi.nlm.nih.gov/BLAST/) and the like, and the specificity of the selected target sequence is confirmed. For example, when AA+(N)19, AA+(N)21 or NA+(N)21 (N is any base) is the target sequence, for the target sequence for which the specificity has been confirmed, a double stranded RNA consisting of a sense strand having a 3'-terminal overhang of TT or UU on 19-21 bases after AA (or NA), and an antisense strand having a sequence complementary to said 19-21 bases and a 3'-terminal overhang of TT or UU, is designed as RNA. In addition, short hairpin RNA (shRNA), which is a precursor of siRNA, can be designed by appropriately selecting any linker sequence (e.g., about 5-25 bases) capable of forming a loop structure and connecting the above-mentioned sense strand and antisense strand via the linker sequence.

siRNA and/or shRNA sequence can be searched for by using a homology search software provided free of charge on various websites. Examples of such site include, but are not limited to, siRNA Target Finder provided by Ambion (http://www.ambion.com/jp/techlib/misc/siRNA_finder.html) and insert design tool for pSilencer™ Expression Vector (http://www.ambion.com/jp/techlib/misc /psilencer_converter.html), GeneSeer provided by RNAi Codex (http://codex.cshl.edu/scripts/newsearchhairpin.cgi).

Examples of siRNA to mRNA of Dnmt-1 gene include, but are not limited to, commercially available siRNAs such as a double stranded RNA consisting of a sense strand composed of the base sequence shown in SEQ ID NO: 9 and an antisense strand shown in SEQ ID NO: 10 (siDnmt-1a in Fig. 8) (Life Technologies Corporation), a double stranded RNA consisting of a sense strand composed of the base sequence shown in SEQ ID NO: 11 and the antisense strand shown in SEQ ID NO: 12 (siDnmt-1b in Fig. 8) (Life Technologies Corporation) and the like.

To improve stability, specific activity and the like, siRNA may be modified in the same manner as in the above-mentioned miRNA.

siRNA can be prepared by synthesizing each of the sense strand and antisense strand of the target sequence on mRNA by a DNA/RNA automatic synthesizer, denaturing them in a suitable annealing buffer at about 90 - about 95°C for about 1 min, and annealing them at about 30 - about 70°C for about 1 - about 8 hrs. It can also be prepared by synthesizing shRNA to be a precursor of siRNA, and cutting same with a dicer.

siRNA can also be introduced into a cell as a precursor designed to be able to produce siRNA to mRNA of Dnmt-1 gene in vivo. As a precursor of siRNA, shRNA can be mentioned. shRNA can be prepared by designing an oligoRNA containing a base sequence formed by connecting a sense strand and an antisense strand of the target sequence on mRNA by inserting, between them, a spacer sequence having a length (e.g., about 5 - 25 bases) capable of forming a suitable loop structure, and synthesizing same by a DNA/RNA automatic synthesizer.

The above-mentioned "nucleic acid containing a base sequence complementary or substantially complementary to the base sequence of mRNA of Dnmt-1 gene or a part thereof" (hereinafter to be also referred to as the nucleic acid of the present invention) may be incorporated into a vector in the form of an expression vector. A vector expressing the nucleic acid of the present invention may be any as long as it is designed to biosynthesize the nucleic acid of the present invention such as miRNA and the like by transcription upon introduction into the cell. For example, the vector expressing siRNA or shRNA includes a tandem type and a stem-loop (hairpin) type. The former includes an expression cassette of the sense strand and an expression cassette of the antisense strand of siRNA connected in tandem, and forms double stranded siRNA (dsRNA) by intracellular expression and annealing of each chain. On the other hand, the latter is a shRNA expression cassette inserted into a vector and forms dsRNA by intracellular expression of shRNA and processing by dicer.

As promoters for such expression vectors, polII promoter (e.g., CMV early-immediate promoter) can be used. For accurate transcription of short RNA, polIII promoter is generally used. Examples of the polIII promoter include mouse and human U6-snRNA promoters, human H1-RNase P RNA promoter, human valine-tRNA promoter and the like. As a transcription termination signal, a sequence containing T continuously in the number of 4 or more is used. Specific examples of the vector capable of intracellularly expressing the nucleic acid of the present invention such as miRNA and the like include pCDNA6.2-GW/miR (manufactured by Invitrogen), pSilencer4.1-CMV (manufactured by Ambion), pSINsi-hH1 DNA (manufactured by Takara Bio Inc.), pSINsi-hU6 DNA (manufactured by Takara Bio Inc.), pENTR/U6 (manufactured by Invitrogen) and the like.

When the above-mentioned substance that suppresses expression of Dnmt-1 (preferably, the nucleic acid of the present invention) is introduced into a cell, lipofection method, liposome method, polyamine method, electroporation method, bead method and the like can be used as an introduction method. A method including forming a lipid complex with a cationic lipid and introducing same into a cell is most general and shows high introduction efficiency. For example, 0.1 - 100 µM, preferably 1 - 10 µM, of nucleic acid can be introduced into a mammalian cell using a commercially available nucleic acid introduction reagent. As an introduction reagent, TransFectin (Bio-Rad), Lipofectamine RNAiMAX (Invitrogen Corporation), HiPerFect Transfection Kit (QUIAGEN), DharmaFEC T reagent kit (Thermo Fisher Scientific) and the like can be used.

A vector expressing the nucleic acid of the present invention can be introduced into a cell by a method known per se according to the kind of the vector. In the case of a virus vector, for example, a plasmid containing a nucleic acid encoding a dominant-negative mutant is introduced into a suitable packaging cell (e.g., Plat-E cell) or a complementary cell line (e.g., 293 cell), a virus vector produced in the culture supernatant is recovered and the cell is infected with the vector by an appropriate method according to each virus vector. In the case of a plasmid vector which is a non-virus vector, the vector can be introduced into a cell by lipofection method, liposome method, electroporation method, calcium phosphate coprecipitation method, DEAE dextran method, microinjection method, particle gun method and the like.

In the present invention, "a substance that inhibits the function of Dnmt-1" may be any as long as an introduction of a methylation state of the parent chain into the daughter chain in the replication process of DNA due to the DNA methyltransferase activity by functionally produced Dnmt-1 is suppressed. Whether the function of Dnmt-1 is inhibited can be confirmed using the aforementioned in vivo or in vitro DNA methyltransferase activity measurement system of Dnmt-1 known per se. As a substance that inhibits the function of Dnmt-1, a substance that inhibits DNA methylation caused by Dnmt-1 can be mentioned. Preferable examples of the substance that inhibits DNA methylation cause by Dnmt-1 include a substance that is incorporated as a nucleotide analogue during DNA synthesis and inhibits methylation, and cytidine analogs such as 5-azacytidine, 5-aza-2'-deoxycytidine (decitabine), 1β-D-ribofuranosyl-2(1H)-pyrimidinone (zebularine) and the like, and a salt thereof, a solvate (e.g., ethanol solvate etc.) thereof, a hydrate thereof, a precursor or derivative (e.g., esterified derivative etc.) thereof can be mentioned. As the substance that inhibits DNA methylation caused by Dnmt-1, non-nucleotide compounds are also known, and preferable examples thereof include a substance that inhibits DNA methylation by masking a Dnmt-1 target sequence (e.g., procaine, procainamide etc.), and a substance that inhibits DNA methylation by blocking the active site of Dnmt-1 (e.g., ((-)-epigallocatechin-3-gallate (EGCG), RG108 etc.). Of the above-mentioned substances that inhibit DNA methylation, RG108 is preferable since it shows a high effect of promoting differentiation into a hepatocyte.

In addition, a substance that inhibits the function of Dnmt-1 may be a substance that inhibits, whether reversibly or irreversibly, the intracellular function of Dnmt-1 by directly or indirectly acting on Dnmt-1. Examples of such substance include a substance that inhibits the intracellular function of Dnmt-1 by inhibiting formation of a complex of Dnmt-1 and other protein (e.g., histone deacetylases (HDAC) etc.), a substance that promotes degradation of Dnmt-1 (e.g., a substance that promotes degradation of Dnmt-1 via proteasome (e.g., 5-aza-2'-deoxycytidine etc.)) and the like.

To be specific, as a substance that inhibits the function of Dnmt-1, for example, a neutralization antibody to Dnmt-1 (i.e., antibody that inhibits the function of Dnmt-1 by binding to Dnmt-1 (e.g., antibody that inhibits binding between Dnmt-1 and HDAC)) can be mentioned. The antibody may be any of a polyclonal antibody and a monoclonal antibody. These antibodies can be selected by screening anti-Dnmt-1 antibodies produced according to the production methods known per se of antibody or anti-serum, for whether or not they inhibit the function of Dnmt-1. While the isotype of antibody is not particularly limited, it is preferably IgG, IgM or IgA, particularly preferably IgG. The antibody is not particularly limited as long as it has at least a complementarity determination region (CDR) for specifically recognize and bind to the target antigen, and it may be a complete antibody molecule or, for example, fragments of Fab, Fab', F(ab')₂ and the like, conjugate molecules produced by genetic engineering such as scFv, scFv-Fc, minibody, diabody and the like, and derivatives thereof which are modified by a molecule having a protein-stabilizing action such as polyethylene glycol (PEG) and the like, and the like.

When the "substance that inhibits the function of Dnmt-1" is a polypeptide, the polypeptide can be introduced into a cell by a method known per se for introducing a protein into a cell. Examples of such method include, but are not limited to, a method using a protein introduction reagent, a method using a protein introduction domain (PTD) or cell permeability peptide (CPP) fusion protein, a microinjection method and the like.

### 2. Method of producing hepatocyte from hepatic progenitor cell or hepatoblastoma

The present invention provides a method of producing a hepatocyte from a hepatic progenitor cell or hepatoblastoma, comprising the following steps (hereinafter to be also referred to as the method of the present invention):
(1) a step of suppressing expression of Dnmt-1 or inhibiting function of Dnmt-1 in a hepatic progenitor cell or hepatoblastoma by contacting the hepatic progenitor cell or hepatoblastoma with the promoter of the present invention (i.e., a substance that suppresses expression of Dnmt-1 or a substance that inhibits the function of Dnmt-1); and
(2) a step of inducing differentiation into a hepatocyte by culturing the hepatic progenitor cell or hepatoblastoma obtained in the aforementioned step (1), wherein expression of Dnmt-1 is suppressed or function of Dnmt-1 is inhibited.

The method of the present invention is based on the promotion of differentiation from hepatic progenitor cell or hepatoblastoma into hepatocyte by the promoter of the present invention, and can produce hepatocyte efficiently. Hepatocyte is a cell as described in the above-mentioned 1. In a preferable embodiment, the hepatocyte produced by the method of the present invention is a hepatocyte with high maturity. In the present invention, the "hepatocyte with high maturity" means a hepatocyte having a high expression level for at least one kind of hepatocyte markers (e.g., albumin, glucose-6-phosphatase (G6pc), tyrosine aminotransferase (Tat), cytochrome P450 (Cyp), miR-122 etc.) (preferably, 4 kinds of albumin, G6pc, Tat and Cyp, more preferably 5 kinds of albumin, G6pc, Tat, Cyp and miR-122), as compared to hepatocytes produced by a method similar to the method of the present invention except that a hepatic progenitor cell or hepatoblastoma without introduction of the promoter of the present invention is used. Preferably, the expression level of a hepatocyte marker is the same as or not less than that of primary culture hepatocyte. The expression level of a hepatocyte marker can be measured by a method known per se, as described in the above-mentioned 1.

The hepatic progenitor cell or hepatoblastoma in step (1) is as described in the above-mentioned 1. The hepatic progenitor cell or hepatoblastoma used in the present invention is generally isolated. In the present invention, being "isolated" means being intentionally placed in a state different from naturally-occurring states. For example, primary cultured cells collected from a living organism and cells induced to differentiate from stem cells are isolated cells.

The contact between a hepatic progenitor cell or hepatoblastoma and the promoter of the present invention (i.e., a substance that suppresses expression of Dnmt-1 or a substance that inhibits the function of Dnmt-1) in step (1) can be performed by, as described in the above-mentioned 1, introducing the substance into a hepatic progenitor cell or hepatoblastoma. When the substance is a nucleic acid, from the aspect of efficiency of introduction into cells, it is preferable to form a lipid complex with a cationic lipid and introduce same into a hepatic progenitor cell or hepatoblastoma, which can be performed, for example, by the following method: using a commercially available nucleic acid introduction reagent (e.g., TransFectin (Bio-Rad) etc.), a lipid complex of the promoter of the present invention and a cationic lipid is formed according to the description in the attached instruction manual. After culture of hepatic progenitor cell or hepatoblastoma for a given time (e.g., about 1 day, 2 days, 3 days, 4 days), the complex is added to a medium to allow contact with the hepatic progenitor cell or hepatoblastoma, and the mixture is further cultured for a given time (e.g., about 5 hrs) to introduce the promoter of the present invention into the hepatic progenitor cell or hepatoblastoma. For culture of the hepatic progenitor cell or hepatoblastoma, a medium widely used for culturing animal cells can be utilized as a basal medium. A commercially available basal medium may also be utilized. Examples thereof include, but are not particularly limited, (minimum essential medium (MEM), Dulbecco's modified minimum essential medium (DMEM), RPMI1640 medium, 199 medium, William's E medium) and the like. These can be used singly, or two or more kinds thereof can be used in combination. Examples of the additive to a medium include various amino acids (e.g., L-glutamine etc.), various inorganic salts, various vitamin, various antibiotics (e.g., penicillin, streptomycin etc.), buffering agents and the like. Generally, introduction of a nucleic acid such as miRNA, siRNA and the like into a cell is performed when the cell is in a logarithmic growth phase (e.g., when cells cover 80% of the bottom surface of the culture container (80% confluent)), it is preferable to add a serum (e.g., fetal bovine serum etc.) to the medium to promote growth of hepatic progenitor cells. Generally, moreover, factors such as growth factor, cytokine, hormone and the like are also added. Examples of the factor include epidermal growth factor (EGF) (about 25 ng/mL), insulin (about 5 µg/mL), hepatocyte growth factor (HGF) (about 25 ng/mL), oncostatin M (about 12.5 ng/mL), hydrocortisone 21-hemisuccinic acid or a salt thereof (about 5×10⁻⁷ M) and dexamethasone (about 10⁻⁷ M). Particularly, EGF is preferably added to the medium to promote growth of hepatic progenitor cells. In a further aspect, a serum (e.g., fetal bovine serum etc.) is preferably added to a medium for growing hepatoblastoma to promote the growth of hepatoblastoma.

When a hepatic progenitor cell or hepatoblastoma is contacted with the promoter of the present invention, it is preferable, from the aspect of introduction efficiency, to exchange a medium suitable for the growth of hepatic progenitor cell or hepatoblastoma as mentioned above (to be also referred to as a medium for hepatic progenitor cell or hepatoblastoma in the present specification) with a special medium used for transfection (also referred to as a medium for transfection) such as OptiMEM (Invitrogen Corporation) and the like.

Since hepatocyte can be obtained efficiently by inducing differentiation into hepatocyte after reaching confluence of hepatic progenitor cell or hepatoblastoma, when the number of cells is small after culture in the aforementioned medium for transfection, the cells may be further cultured in a medium for hepatic progenitor cell or hepatoblastoma. Such additional culture in the medium for hepatic progenitor cell or hepatoblastoma may be performed until the cells reach confluence. Generally, the total culture time in the medium for hepatic progenitor cell or hepatoblastoma, including the culture time in the medium for hepatic progenitor cell or hepatoblastoma before contact with the promoter of the present invention, is not less than 3 days (about 4 days).

In addition, the contact between a hepatic progenitor cell or hepatoblastoma and the promoter of the present invention (i.e., a substance that suppresses expression of Dnmt-1 or a substance that inhibits the function of Dnmt-1) can be performed by culturing hepatic progenitor cell or hepatoblastoma in the presence of the promoter of the present invention. Examples of the medium include the above-mentioned medium used for introducing the promoter of the present invention into a hepatic progenitor cell or hepatoblastoma, and a medium for hepatic progenitor cell or hepatoblastoma. While the contact conditions are appropriately determined, for example, pH is about 6 - about 8, and the temperature is generally about 30 - about 40°C. The contact time and the concentration of the promoter of the present invention on contact are not particularly limited as long as expression of Dnmt-1 is suppressed or the function of Dnmt-1 is inhibited in the hepatic progenitor cell or hepatoblastoma, and can be appropriately determined according to the active ingredient of the promoter of the present invention. For example, when 5-aza-2'-deoxycytidine (decitabine) is used as the promoter of the present invention, hepatic progenitor cells are cultured in the medium for hepatic progenitor cell, and the promoter of the present invention at a concentration of about 0.2 - 1.0 µM is brought into contact therewith from day 2 of culture until the cells become confluent. In a further aspect, they may be contacted with hepatoblastoma from day 0 of culture until the cells become confluent in the medium for hepatoblastoma added with 5-aza-2'-deoxycytidine (decitabine) at a concentration of 2.5-20 µM. For example, when RG108 is used as the promoter of the present invention, hepatic progenitor cells are cultured in the medium for hepatic progenitor cell, and the promoter of the present invention at a concentration of about 20 - 100 µM is brought into contact therewith from day 2 of culture until the cells become confluent. In a further aspect, they may be contacted with hepatoblastoma from day 0 of culture until the cells become confluent in the medium for hepatoblastoma added with RG108 at a concentration of about 20-100 µM. When hepatic progenitor cell or hepatoblastoma is contacted with the promoter of the present invention by culturing hepatic progenitor cell or hepatoblastoma in the presence of the promoter of the present invention, the culture in the below-mentioned step (2) is also preferably performed in the presence of the promoter of the present invention to more efficiently promote differentiation into a hepatocyte. In this case, the concentration of the promoter of the present invention only needs to be of the same level as in step (1).

The culture in step (2) can be performed by any method as long as induction of differentiation from hepatic progenitor cell into hepatocyte is possible, and a known method can be used as a method for differentiation induction into hepatocyte. Examples of the known method include a method of culturing in a culture medium added with oncostatin M, dexamethasone, hepatocyte growth factor (HGF) and the like (Journal of Cellular Physiology, Vol. 227(5), p.2051-2058 (2012); Hepatology, Vol.45(5), p.1229-1239 (2007)) and the like. As a differentiation induction method from hepatoblastoma into hepatocyte, a method of culturing continuously in a culture medium and the like can be mentioned.

For culture in step (2), a medium widely used for culturing animal cells can be utilized as a basal medium (also referred to as basal medium for hepatocyte in the present specification). A commercially available basal medium may also be utilized. Examples thereof include, but are not particularly limited, (minimum essential medium (MEM), Dulbecco's modified minimum essential medium (DMEM), RPMI1640 medium, 199 medium, William's E medium) and the like. These can be used singly, or two or more kinds thereof can be used in combination. Examples of the additive to a medium include various amino acids (e.g., L-glutamine etc.), various inorganic salts, various vitamin, various antibiotics (e.g., penicillin, streptomycin etc.), buffering agents and the like.

For differentiation induction from hepatic progenitor cell into hepatocyte, generally, factors such as growth factor, cytokine, hormone and the like are also added to the above-mentioned basal medium for hepatocyte. As long as a basal medium for hepatocyte added with these factors (also referred to as differentiation induction medium in the present specification) can induce differentiation from hepatic progenitor cell into hepatocyte, the factors to be added are not particularly limited. For example, as described in the below-mentioned Examples, differentiation induction from hepatic progenitor cell into hepatocyte can be performed by using a medium added with, as these factors, insulin (about 5 µg/mL), hepatocyte growth factor (HGF) (about 25 ng/mL), oncostatin M (about 12.5 ng/mL), hydrocortisone 21-hemisuccinic acid or a salt thereof (about 5×10⁻⁷ M) and dexamethasone (about 10⁻⁷ M). For example, as disclosed in JP-A-2000-287680, differentiation induction from immature hepatocyte into hepatocyte is also possible by using oncostatin M and dexamethasone from among the above-mentioned factors. To grow hepatic progenitor cells, a medium added with EGF and serum (e.g., fetal bovine serum etc.) is used; however, use of a medium free of these is preferable for differentiation induction.

For differentiation induction from hepatoblastoma into hepatocyte, a serum (e.g., fetal bovine serum etc.) is generally further added to the above-mentioned basal medium. As long as a medium for hepatoblastoma added with these factors (also referred to as medium for hepatoblastoma in the present specification) can induce differentiation from hepatoblastoma into hepatocyte, the factors to be added are not particularly limited.

In one embodiment, regarding EGF and serum in the above-mentioned basal medium for hepatocyte (differentiation induction medium), a medium free of EGF and having a decreased serum concentration can also be used. The amount of the reduced serum may be 0.5 - 2%(w/w), preferably 1%(w/w).

Differentiation of hepatic progenitor cell or hepatoblastoma obtained in step (1) wherein the expression of Dnmt-1 is suppressed or the function of Dnmt-1 is inhibited can be induced by culturing same in a differentiation induction medium or a medium for hepatoblast. The culture conditions are not particularly limited as long as differentiation from hepatic progenitor cell or hepatoblastoma into hepatocyte is induced and are appropriately determined. For example, the pH of the medium is about 6 - about 8 and the culture temperature is generally about 30 - about 40°C. Also, the culture time is not particularly limited, and can be appropriately determined according to the method of differentiation induction into hepatocyte.

In the above-mentioned steps (1) and (2), differentiation from hepatic progenitor cell or hepatoblastoma into hepatocyte is promoted and the hepatocyte can be produced efficiently. Production of hepatocyte can be confirmed using expression of a hepatocyte marker as an index. The expression level of a hepatocyte marker can be measured by a method known per se, as described in the above-mentioned 1. Hepatocyte generally has functions such as glucose production capacity, ammonia metabolic capacity, albumin production capacity, urea synthesis ability and the like. Therefore, production of hepatocyte can also be confirmed using the presence or absence of these functions as an index. The glucose production capacity can be confirmed by analyzing the glucose level of culture supernatant by a glucose oxidase method. The ammonia metabolic capacity can be confirmed by analyzing the ammonia level of culture supernatant by a modified indophenol method (Horn DB & Squire CR, Clin. Chim. Acta. 14(2): p.185-94 (1966)). The albumin production capacity can be confirmed by analyzing the albumin concentration of culture supernatant by a method of measuring the serum albumin concentration. In addition, the urea synthesis ability can be confirmed, for example, by using colorimetric assay (Sigma Ltd.). In a preferable embodiment of the present invention, production of hepatocyte having high maturity can be confirmed by measuring the expression level of a hepatocyte marker and comparing said level with that of control (expression level when differentiation induction of hepatic progenitor cell or hepatoblastoma without introduction of the promoter of the present invention is performed in the same manner).

According to the method of the present invention, hepatocyte used for the prediction of hepatotoxicity and liver metabolite, and the like can be supplied stably. In a preferable embodiment of the present invention, since a hepatocyte with high maturity as compared to hepatocytes produced by a conventional method can be produced, hepatocyte more preferably used for the prediction of hepatotoxicity and liver metabolite, and the like can be supplied stably. Therefore, the method of the present invention is useful in the fields of, for example, development of pharmaceutical products, food and the like.

When a substance having an activity to suppress expression of Dnmt-1, or inhibit the function of Dnmt-1 activity is added to a medium for differentiation induction of hepatocyte, the substance may show cytotoxicity. Therefore, when differentiation into hepatocyte is induced using the promoter of the present invention, the cytotoxicity can be reduced by seeding at high cell density. Preferable cell density at seeding is, for example, at 4×10⁴ - 2×10⁵ cells/cm².

Particularly, when differentiation induction is performed for a long term, seeding at the above-mentioned cell density is preferable to suppress cytotoxicity of the substance having an activity to suppress expression of Dnmt-1, or inhibit the function of Dnmt-1.

In a preferable embodiment of the present invention, the culture in the above-mentioned step (2) is performed for a long term. A long-term differentiation induction affords hepatocyte with more advanced maturity. In such cell, an increase in the expression of a hepatocyte marker is induced in not only the mRNA level but also protein level. A preferable period of long-term differentiation induction is not particularly limited as long as the expression of a hepatocyte marker at a protein level is induced. For example, not less than 7 days, not less than 10 days, not less than 13 days and the like can be mentioned.

To confirm expression induction of hepatocyte marker at a protein level in hepatocyte with more advanced maturity, the protein amount of the above-mentioned hepatocyte marker can be quantified by a method known per se such as Western blotting and the like, or for example, the activity of CYP enzymes (e.g., CYP1A2 and CYP3A4 etc.) can also be measured.

Alternatively, hepatocyte with more advanced maturity has a specific morphology with multiple nuclei, clear cell boundary under a microscope and the like, and/or may show promoted glycogen storage capacity. Whether the cell has multiple nuclei can be determined by a confirmation method using an optical microscope, a method of staining the nucleus with a known nuclear staining agent (e.g., DAPI, Hoechst33342 etc.) and the like, and clear cell boundary can be confirmed by an optical microscope. In addition, glycogen storage capacity can be confirmed by a method known to those of ordinary skill in the art such as PAS staining and the like. In a preferable embodiment, hepatocyte with advanced maturity, which is induced by long-term culture, expresses CYP1A2 and CYP3A4 proteins, has multiple nuclei and PAS staining positive.

The present invention also provides hepatocyte produced by the method of the present invention. The hepatocyte produced by the method of the present invention can be utilized, for example, for the evaluation of metabolism and hepatotoxicity of a test compound and the like.

For the evaluation of metabolism and hepatotoxicity of a test compound, animal model and the like have conventionally been used. However, the number of test compounds that can be evaluated at one time is limited, and evaluation obtained in animal model and the like cannot be directly applied to human. Therefore, an evaluation method using human liver cancer cell line and primary normal human cultured hepatocyte has been adopted. However, since human liver cancer cell line is a cancer cell, the evaluation obtained in human liver cancer cell line may not be applicable to human normal hepatocyte. In addition, primary normal human cultured hepatocyte poses problems in terms of stable supply and cost. Moreover, a cell line immortalizing primary normal human cultured hepatocyte shows decreased CYP3A4 activity as compared to the absence of immortalization (International Journal of Molecular Medicine 14: 663-668, 2004, Akiyama I. et al.). Such problems can be solved by utilizing hepatocyte produced by the method of the present invention.

Accordingly, the present invention further provides a method of evaluating the metabolism of a test compound. In the method, hepatocyte produced by the method of the present invention is contacted with a test compound. Then, the metabolism of the test compound contacted with the hepatocyte is measured.

The test compound to be used in the present invention is not particularly limited. Examples thereof include, but are not limited to, single compounds such as xenobiotic substance, natural compound, organic compound, inorganic compound, protein, peptide and the like, and expression products of compound library and gene library, cell extract, cell culture supernatant, fermentation microorganism product, marine organism extract, plant extract and the like.

Examples of the xenobiotic substance include, but are not limited to, candidate compounds of medicament and food, existing medicament and food, and any substance foreign to living organisms is included in the xenobiotic substance in the present invention. More specifically, Rifampin, Dexamethasone, Phenobarbital, Ciglirazone, Phenytoin, Efavirenz, Simvastatin, β-Naphthoflavone, Omeprazoie, Clotrimazole, 3-Methylcholanthrene and the like can be recited as examples.

The "contact" in the present invention is generally made by adding a test compound to a medium or a culture medium, but the method is not limited thereto. When the test compound is a protein and the like, the "contact" can be made by introducing a DNA vector expressing the protein into the cell.

The metabolism of a test compound can be measured by a method well known to those of ordinary skill in the art. For example, when a metabolite of the test compound is detected, the test compound is judged to have been metabolized. In addition, when the expression of enzyme genes such as CYP (cytochrome p450), MDR, MPR and the like is induced, or the activity of these enzymes increased by contact with the test compound, the test compound is judged to have been metabolized.

Also, the present invention provides an evaluation method of hepatotoxicity of a test compound. In the method, hepatocyte produced by the method of the present invention is contacted with a test compound. Then, the level of disorder in the hepatocyte contacted with the test compound is measured. The level of disorder can be measured, for example, by using a survival rate of the hepatocyte or hepatopathy markers such as GOT, GPT and the like as an index.

For example, when the survival rate of hepatocyte decreases by the addition of a test compound to the culture medium of the hepatocyte, the test compound is judged to have hepatotoxicity, and when the survival rate does not change significantly, the test compound is judged to have no hepatotoxicity. For example, when GOT and GPT in the culture medium of hepatocyte increase after addition of a test compound, the test compound is judged to have hepatotoxicity, and when GOT and GPT do not show a significant change, the test compound is judged to have no hepatotoxicity.

When a compound having determined hepatotoxicity is used as a control, the presence or absence of hepatotoxicity in the test compound can be evaluated more accurately.

### Examples

The Examples of the present invention are explained in the following, which are not to be construed as limitative.

### Materials and methods

### (1) Recovery of hepatic tissue

### (i) Preparation of HEPES buffer

To autoclave-sterilized distilled water (750 mL) were added 8 g of sodium chloride (Sigma-Aldrich Corporation), 0.2 g of potassium chloride (Sigma-Aldrich Corporation), 0.1 g of sodium hydrogenphosphate dodecahydrate (Wako Pure Chemical Industries, Ltd.) and 10 mL of HEPES solution (2.38 g/10 mL, Invitrogen Corporation), and the mixture was adjusted to 1 L with stirring. To the obtained HEPES buffer (100 mL) was added 56.25 mg of calcium chloride dihydrate (Sigma-Aldrich Corporation) to prepare 100 mL of HEPES buffer (containing calcium).

### (ii) Recovery of mouse embryo-derived hepatic tissue

Two pregnant mice (E14.5) were anesthetized and sacrificed by cervical dislocation. Abdominal cavity was opened with surgical scissors, and uterus containing embryo was carefully removed and transferred into ice-cooled PBS solution (Sigma-Aldrich Corporation: D8537). The embryo was removed with tweezers and transferred into a 10 cm petri dish containing HEPES buffer. Using stereoscopic microscope, the embryo was anatomize and hepatic tissue was recovered. The recovered hepatic tissue was transferred into the petri dish containing HEPES buffer, hepatic capsule was carefully removed to give a hepatic tissue, which was used for collection of hepatic parenchymal cells.

### (2) Collection of hepatic parenchymal cell

### (i) Preparation of enzyme solution

To 5 mg of freeze-dried liberase (Roche) was added 2 mL of distilled water, and the mixture was left standing in ice for 30 min, and stirred every 2-3 minutes until complete dissolution to prepare 13 U/mL of liberase solution. The 13 U/mL of liberase solution was used as a stock solution and preserved at -20°C. When in use, 100 µL of liberase solution (13 U/mL) was added to 10 mL of HEPES buffer (containing calcium) to prepare an enzyme solution.

### (ii) Preparation of basal medium for hepatocyte

To William's E medium (Invitrogen Corporation) were added 2 mM of L-glutamine solution (Invitrogen Corporation), 50 U/mL of penicillin - 50 µg/mL of streptomycin solution (Invitrogen Corporation) and 10% fetal bovine serum (Thermo Fisher Scientific K.K.) to prepare the medium.

### (iii) Collection of embryo hepatic tissue-derived hepatic parenchymal cells

The recovered hepatic tissue was transferred into a 1.5 mL tube, and thoroughly cut into small pieces with surgical scissors. An enzyme treatment was performed by the following procedures. The minced tissue was placed in a 50 mL tube, suspended in 10 mL of enzyme solution warmed to 37°C, and incubated at 37°C for 5 min, during which the tube was stirred every 2-3 minutes. Thereafter, 25 mL of the basal medium for hepatocyte was added to a 2 mL plastic pipette, and the enzyme reaction was quenched by diluting the enzyme solution. The diluted enzyme solution containing the cells was filtered with a 60 µm nylon filter to give a cell suspension. Subsequently, the filter was washed with the basal medium for hepatocyte, and the medium was recovered in the same container as the cell suspension. The cell suspension was adjusted to about 100 mL. The cell suspension was transferred into Erlenmeyer flask, and left standing at room temperature for 15 min. After standing, the upper layer (three-quarters of total volume) was removed with a 25 mL plastic pipette. To the remaining cell suspension was added 25 mL of the basal medium for hepatocyte, and the mixture was suspended again. The suspension was divided into two 50 mL round-bottom tubes with a 25 mL plastic pipette, the basal medium for hepatocyte was added to each tube, and the volume was adjusted to 40 mL. The mixture was centrifuged at 4°C, 1000 rpm (22×g) for 2 min, and the supernatant was removed to give hepatic parenchymal cells.

### (3) Isolation of hepatic progenitor cell

### (i) Preparation of medium for hepatic progenitor cell

To a basal medium for hepatocyte were added 5 µg/mL of insulin (Sigma-Aldrich Corporation), 25 ng/mL of epidermal growth factor (EGF) (Sigma-Aldrich Corporation), 25 ng/mL of hepatocyte growth factor (HGF) (Peprotech), 12.5 ng/mL of oncostatin M (Sigma-Aldrich Corporation), 5×10⁻⁷M hydrocortisone 21-hemisuccinate sodium salt (Sigma-Aldrich Corporation) and 10⁻⁷M dexamethasone (Sigma-Aldrich Corporation) to prepare the medium.

### (ii) Isolation of hepatic progenitor cell

The hepatic parenchymal cells were diluted with HEPES buffer added with 2% fetal bovine serum to prepare a 10⁷ cells/100 µL cell suspension, which was transferred into a 5 mL round-bottom tube. A mouse FcR blocking antibody in EasySep Mouse Biotin Positive Selection Kit (STEMCELL Technologies Inc) was added at 1 µL per 10⁷ cells, and the mixture was admixed by tapping the tube. Biotin-labeled anti-E-cadherin antibody (eBioscience) was added at 2-4 µL per 10⁷ cells, and the mixture was left standing at room temperature for 15 min while stirring the tube every 2-3 minutes. Biotin Selection Cocktail in EasySep Mouse Biotin Positive Selection Kit was added at 10 µL per 10⁷ cells, and the mixture was left standing at room temperature for 15 min with occasional stirring. Magnetic particles in EasySep Mouse Biotin Positive Selection Kit were added at 5 µL per 10⁷ cells, and the mixture was stirred and left standing at room temperature for 10 min. To the cell suspension was added a medium for cell isolation, and the volume was adjusted to 2.5 mL. The mixture was suspended with a 2 mL plastic pipette, and the tube was placed in an exclusive magnet (STEMCELL Technologies Inc.) and left standing at room temperature for 10 min. The exclusive magnet containing the tube was reversed for 3 sec, and a non-hepatic progenitor cell fraction was removed (purification operation: first time). The tube was taken out from the exclusive magnet, 2.5 mL of the medium for cell isolation was added, and a purification operation was performed again (purification operation: second time). Furthermore, 2.5 mL of the medium for cell isolation was added, and a purification operation was performed for the third time (purification operation: third time). The cells remaining in the tube are the hepatic progenitor cells.

### (4) Culture of liver cancer cell line

Mouse liver cancer-derived cell line (Hepal-6) and human liver cancer-derived cell lines (Huh-7, HepG2, Hep3B) were cultured in DMEM (Invitrogen Corporation) added with 50 U/mL penicillin - 50 µg/mL streptomycin solution (Invitrogen Corporation) and 10% fetal bovine serum (Thermo Fisher Scientific K.K.).

### (5) Extraction of total RNA

Total RNA was extracted from cultured cells, liver cancer cell lines, and mouse and human normal hepatic tissues by using miRNeasy Mini Kit (QUIAGEN). The total RNA was quantified by NanoDrop 1000 spectrophotometer (Thermo Fisher Scientific K.K.). The hepatic tissues recovered from mouse were placed in ice-cooled phosphate buffer and disrupted before extraction of RNA. Red blood cells were lysed in Red Blood Cell Lysis Buffer (Sigma-Aldrich Corporation).

### (6) Quantification by the real-time PCR method

RNA was quantified by the real-time PCR method using 7300 real-time PCR system (Applied Biosystems) and TaqMan MicroRNA Assays (Applied Biosystems). The miRNA expression was standardized at 6NU6B level. For quantification of messenger RNA, total RNA sample was treated with DNase (TURBO DNA-free kit: Ambion Inc.), and cDNA was synthesized from 1 µg of total RNA by using reverse transcriptase (SuperScript III Reverse Transcriptase: Ambion Inc.). For quantification, SYBR Green (PlatinumSYBR Green quantitative PCR SuperMix UDG: Invitrogen Corporation) was used. The reaction for real-time PCR was performed at 95°C, 2 min, 40 cycles (95°C for 15 sec, 60°C for 30 sec). The cDNA expression level was standardized by GAPDH.

### (7) Introduction of miRNA precursor or siRNA into cell

For introduction of miRNA precursor into liver cancer cell line, the cells were plated on a 35 mm dish for cell culture at 4×10⁵ cells and, after 24 hr of culture, 100 nM miRNA precursor and 5 µL of a transfectin reagent (Bio-Rad) were added to 1 mL of OptiMEM (Invitrogen Corporation). miRNA precursor was introduced into hepatic progenitor cells on day 4 of culture by a method similar to that of miRNA precursor into liver cancer cell line. siRNA was introduced into hepatic progenitor cell on day 2 of culture by a method similar to that of miRNA precursor into liver cancer cell line and using 100 nM siRNA (two kinds).

### (8) Culture of human hepatic progenitor cell

Human hepatic progenitor cells (Cell Systems Corporation) were cultured in DMEM (Invitrogen Corporation) added with 10% fetal bovine serum (MP Bio).

### (9) Extraction of total RNA

Total RNA was extracted from human hepatic progenitor cell by using miRNeasy Mini Kit (QUIAGEN). Total RNA was quantified by Synergy H1-Take3 plate (BIOTEC Co., Ltd.).

### (10) Quantification by the real-time PCR method

RNA was quantified by the real-time PCR method and using StepOne real-time PCR system (Applied Biosystems) and TaqMan Gene Expression Assays (Applied Biosystems). For quantification of messenger RNA, cDNA was synthesized from 1 µg of total RNA by using reverse transcriptase (ReverTra Ace qPCR RT Master Mix: TOYOBO CO., LTD.). For quantification, TaqMan Universal PCR Master Mix, No AmpErase UNG (Invitrogen Corporation) was used. The reaction for real-time PCR was performed at 50°C, 2 min and 95°C, 10 min, 40 cycles (95°C for 15 sec, 60°C for 1 min). The cDNA expression level was standardized by 18S ribosomal RNA.

### (11) Introduction of miRNA precursor into human hepatic progenitor cell

For introduction of miRNA precursor into human hepatic progenitor cell, the cells were plated on a 6-well plate at 2×10⁴ cells/cm² and, at 80% confluent, 20 nM miRNA precursor and 3.7 µL of a transfectin reagent (Bio-Rad) were added to 1.25 mL of OptiMEM (Invitrogen Corporation).

### Example 1 Differentiation and maturation of hepatic progenitor cell

Mouse hepatic progenitor cells were plated at 2×10⁶ cells/cm² on a 35 mm cell culture dish coated with type I collagen, and cultured in a medium for hepatic progenitor cell. After confluence, the cells were cultured in a medium for hepatic progenitor cell free of epidermal growth factor (EGF) and fetal bovine serum. The medium was exchanged every day.

The morphological changes of the cells are shown in Fig. 1. The cells became confluent in 3 days of culture and the morphology of the cells was quadrate (Fig. 1a). The cell morphology became oval after 5 days of culture (Fig. 1b). On day 12 of culture, the cells showed morphology of matured hepatocyte (Figs. 1c and d).

### Example 2 Quantification of miR-148a in human and mouse hepatic tissues or liver cancer-derived cells

miR-148a expressed in mouse liver cancer-derived cell line Hepa1-6 (American Type Culture Collection: ATCC), human liver cancer-derived cell lines HepG2, Hep3B (American Type Culture Collection: ATCC), human liver cancer-derived cell line Huh-7 (RIKEN cell bank), mouse (embryo mouse, newborn mouse, adult mouse) hepatic tissues (collected by a method similar to that in the above-mentioned (1) and (2)) and human normal hepatic tissue (obtained from National Cancer Center) was quantified by the real-time PCR method. As shown in Fig. 2, miR-148a was highly expressed only in adult mouse hepatic tissue and human normal hepatic tissue.

### Example 3 Changes in miR-148a expression level after differentiation induction of hepatic progenitor cell

Mouse hepatic progenitor cells were plated at 4×10⁴ cells/cm² in a 35 mm cell culture dish coated with type I collagen, and cultured in a medium for hepatic progenitor cells. After confluence (after day 4 of culture), the cells were cultured in the medium for hepatic progenitor cells free of epidermal growth factor (EGF) and fetal bovine serum. The medium was exchanged every day. Total RNA containing miRNA was extracted, and miR-148a was quantified by the real-time PCR method.

As shown in Fig. 3, the miR-148a expression level increased with maturation of the hepatic progenitor cells after differentiation induction.

### Example 4 Changes in miR-148a expression level after differentiation induction of hepatic progenitor cell introduced with miR-148a precursor

Mouse hepatic progenitor cells were plated at 4×10⁴ cells/cm² in a 35 mm cell culture dish coated with type I collagen, and cultured in a medium for hepatic progenitor cells. miR-148a precursor was introduced into the cultured mouse hepatic progenitor cells and, after confluence (after day 4 of culture), the cells were cultured in the medium for hepatic progenitor cells free of epidermal growth factor (EGF) and fetal bovine serum. The medium was exchanged every day. Total RNA containing miRNA was extracted, and miR-148a was quantified by the real-time PCR method. When miR-148a precursor was not introduced, the measurement was started from day 2 of culture; when miR-148a precursor was introduced, the measurement was started from day 5 of culture after introduction of miRNA.

As shown in Fig. 4, miR-148a was overexpressed from day 5 of culture after introduction of miRNA.

### Example 5 Changes in expression level of hepatocyte marker after differentiation induction

Mouse hepatic progenitor cells were plated at 4×10⁴ cells/cm² in a 35 mm cell culture dish coated with type I collagen, and cultured in a medium for hepatic progenitor cells. miR-148a precursor was introduced into the cultured mouse hepatic progenitor cells and, after confluence (after day 4 of culture), the cells were cultured in the medium for hepatic progenitor cells free of epidermal growth factor (EGF) and fetal bovine serum. Total RNA was extracted, and the expression levels of genes of albumin, glucose-6-phosphatase (G6pc) present in hepatocyte, tyrosine amino group transferase (Tat), drug-metabolizing enzyme (Cyp17a1), which are hepatocyte marker, and miR-122 which is liver-specifically expressed were quantified by the real-time PCR method. When miR-148a precursor was not introduced, the measurement was started from day 2 of culture; when miR-148a precursor was introduced, the measurement was started from day 5 of culture after introduction of miRNA.

As shown in Fig. 5, the expression levels of albumin, G6pc, Tat, Cyp17a1 and miR-122 all increased after differentiation induction. When miR-148a precursor was introduced, high expression levels were observed from day 5 of culture, as compared to no introduction of miR-148a precursor.

### Example 6 Changes in Dnmt-1 expression level after differentiation induction

Mouse hepatic progenitor cells were plated at 4×10⁴ cells/cm² in a 35 mm cell culture dish coated with type I collagen, and cultured in a medium for hepatic progenitor cells. After confluence (after day 4 of culture), the cells were cultured in the medium for hepatic progenitor cells free of epidermal growth factor (EGF) and fetal bovine serum. The medium was exchanged every day. Total RNA was extracted, and Dnmt-1 was quantified by the real-time PCR method.

As shown in Fig. 6, the Dnmt-1 expression level decreased after differentiation induction, contrary to the miR-148 expression level.

### Example 7 Changes in Dnmt-1 expression level after differentiation induction of hepatic progenitor cell introduced with miR-148a precursor

Mouse hepatic progenitor cells were plated at 4×10⁴ cells/cm² in a 35 mm cell culture dish coated with type I collagen, and cultured in a medium for hepatic progenitor cells. miR-148a precursor was introduced into the cultured mouse hepatic progenitor cells and, after confluence (after day 4 of culture), the cells were cultured in the medium for hepatic progenitor cells free of epidermal growth factor (EGF) and fetal bovine serum. The medium was exchanged every day. Total RNA was extracted, and Dnmt-1 was quantified by the real-time PCR method.

As shown in Fig. 7, the Dnmt-1 expression levels on days 4 and 5 of culture further decreased when miR-148a precursor was introduced, as compared to no introduction of miR-148a precursor. The results show that introduction of miR-148a can suppress the expression of Dnmt-1 to a lower level in differentiation induction into hepatocyte.

### Example 8 Changes in expression level of hepatocyte marker after differentiation induction, due to inhibition of Dnmt-1 expression

Mouse hepatic progenitor cells were plated at 4×10⁴ cells/cm² in a 35 mm cell culture dish coated with type I collagen, and cultured in a medium for hepatic progenitor cells. siRNA that suppresses expression of Dnmt-1 (siDnmt-1a or b mentioned below) was introduced into the cultured mouse hepatic progenitor cells and, after confluence (after day 4 of culture), the cells were cultured in the medium for hepatic progenitor cells free of epidermal growth factor (EGF) and fetal bovine serum. On day 6 of culture, total RNA was extracted. The expression level of Dnmt-1, and the expression level of genes of albumin, glucose-6-phosphatase (G6pc) present in hepatocyte, tyrosine amino group transferase (Tat), and drug-metabolizing enzyme (Cyp17a1), which are hepatocyte marker, were quantified by the real-time PCR method. The expression levels of genes of hepatocyte marker albumin, glucose-6-phosphatase present in hepatocyte and drug-metabolizing enzyme (G6pc, Tat and Cyp) were quantified by the real-time PCR method.
siDnmt-1a:
sense strand: GGUAGAGAGUUACGACGAAtt (SEQ ID NO: 9)
antisense strand: UUCGUCGUAACUCUCUACCtg (SEQ ID NO: 10) siDnmt-1b:
sense strand: CAACGGAUCCUAUCACACUtt (SEQ ID NO: 11)
antisense strand: AGUGUGAUAGGAUCCGUUGta (SEQ ID NO: 12)

As shown in Fig. 8, when siDnmt-1a or b was introduced, high expression levels were found in either gene as compared to introduction of control siRNA (siCtrl) that does not suppress expression of Dnmt-1. In addition, the expression of Dnmt-1 was suppressed as compared to introduction of control siRNA (siCtrl).

### Example 9 Changes in expression level of miR-148a after differentiation induction, due to substance that inhibits DNA methylation caused by Dnmt-1

Mouse hepatic progenitor cells were plated at 1×10⁵ cells/cm² in a 35 mm cell culture dish coated with type I collagen, and cultured in a medium for hepatic progenitor cells. On day 2 of culture, the medium was exchanged with a medium for hepatic progenitor cell, containing a substance that inhibits DNA methylation caused by Dnmt-1 (5-aza-2'-deoxycytidine (decitabine): 0.2 µM or 1.0 µM, or RG108: 20 µM or 100 µM), and the mouse hepatic progenitor cells were cultured to confluence. After confluence, the cells were cultured in the medium for hepatic progenitor cells free of epidermal growth factor (EGF) and fetal bovine serum but added with a substance that inhibits DNA methylation (5-aza-2'-deoxycytidine (decitabine): 0.2 µM (5-aza 0.2) or 1.0 µM (5-aza 1.0), or RG108: 20 µM (RG 20) or 100 µM (RG 100)), whereby differentiation was induced. The medium was exchanged every day. On day 7 of culture, total RNA was extracted, and the miR-148a expression level was quantified by the real-time PCR method.

As shown in Fig. 9, the expression of miR-148a showed a high level when differentiation was induced in a medium containing a substance that inhibits DNA methylation caused by Dnmt-1, as compared to differentiation induction in a medium without addition of a substance that inhibits DNA methylation caused by Dnmt-1 (control).

### Example 10 Changes in expression level of hepatocyte marker after differentiation induction, due to substance that inhibits DNA methylation caused by Dnmt-1

Mouse hepatic progenitor cells were plated at 1×10⁵ cells/cm² in a 35 mm cell culture dish coated with type I collagen, and cultured in a medium for hepatic progenitor cells. On day 2 of culture, the medium was exchanged with a medium for hepatic progenitor cell, containing a substance that inhibits DNA methylation caused by Dnmt-1 (5-aza-2'-deoxycytidine (decitabine): 0.2 µM or 1.0 µM, or RG108: 20 µM or 100 µM), and the mouse hepatic progenitor cells were cultured to confluence. After confluence, the cells were cultured in the medium for hepatic progenitor cells free of epidermal growth factor (EGF) and fetal bovine serum but added with a substance that inhibits DNA methylation (5-aza-2'-deoxycytidine (decitabine): 0.2 µM or 1.0 µM, or RG108: 20 µM or 100 µM), whereby differentiation was induced. The medium was exchanged every day. On day 7 of culture, total RNA was extracted, and the expression levels of hepatocyte marker albumin, a drug-metabolizing enzyme (Cyp3a4), and miR-122 that expresses liver-specifically were quantified by the real-time PCR method.

As shown in Figs. 10B and C, the expression of both Cyp3a4 and miR-122 showed a high level when differentiation was induced in a medium containing a substance that inhibits DNA methylation caused by Dnmt-1, as compared to differentiation induction in a medium without addition of a substance that inhibits DNA methylation caused by Dnmt-1 (control). As shown in Fig. 10A, moreover, the expression of albumin showed a high level when differentiation was induced in a medium containing RG108, as compared to the control. These results show that differentiation into hepatocyte is promoted when differentiation is induced in a medium containing a substance that inhibits DNA methylation caused by Dnmt-1.

### Example 11 Changes in expression level of hepatocyte marker after differentiation induction in hepatic progenitor cell introduced with miR-148a precursor

Human hepatic progenitor cells (Cell System Corporation) were plated at 2×10⁴ cells/cm² on a 6-well plate coated with matrigel (Becton, Dickinson and Company) and cultured. miR-148a precursor was introduced into the cultured human hepatic progenitor cells and, after confluence, the cells were cultured in a medium for hepatic progenitor cell, which contained fetal bovine serum lowered to 1%. The medium was exchanged every day. Total RNA was extracted on days 5, 7 and 9 after miR-148a precursor introduction. The expression levels of the genes of albumin and the drug-metabolizing enzyme (Cyp3A4) which are hepatocyte markers were quantified by the real-time PCR method.

As shown in Fig. 11, the expression levels of albumin and Cyp3A4 showed a high level when miR-148a precursor was introduced, as compared to introduction of miRNA (miCtrl) that does not have function and culture in a medium alone (control).

### Example 12 Morphology of hepatic progenitor cell introduced with miR-148a precursor

Human hepatic progenitor cells (Cell System Corporation) were plated at 2×10⁴ cells/cm² on a 6-well plate coated with matrigel (Becton, Dickinson and Company) and cultured. miR-148a precursor was introduced into the cultured human hepatic progenitor cells and, after confluence, the cells were cultured in a medium for hepatic progenitor cell, which contained fetal bovine serum lowered to 1%. The medium was exchanged every day. The morphology on day 14 of culture is shown in Fig. 12. A cell having morphology specific to mature hepatocyte (multiple nuclei, clear cell boundary) was observed.

From the above results, it was shown that miR-148a promotes differentiation of hepatic progenitor cell by suppressing expression of Dnmt-1 and achieves differentiation into hepatocyte having high maturity.

### Example 13 Morphology of HepG2 cell cultured in medium added with substance that inhibits DNA methylation caused by Dnmt-1

Hepatoblastoma HepG2 cells (American Type Culture Collection: ATCC) were plated on a 35 mm cell culture dish at 7×10⁵ cells, and cultured in DMEM (Invitrogen Corporation) added with 100 IU/mL penicillin - 100 µg/mL streptomycin solution (Invitrogen Corporation), 10% fetal bovine serum (Thermo Fisher Scientific K.K.), and a substance that inhibits DNA methylation (5-aza-2'-deoxycytidine: 5-aza (decitabine): 2.5 µM). The medium was exchanged every day. The morphology on day 14 of culture is shown in Fig. 13. HepG2 cells cultured in a medium added with a DNA methylation inhibitor had morphology specific to mature hepatocyte (multiple nuclei, clear cell boundary).

### Example 14 PAS staining of HepG2 cell cultured in medium added with substance that inhibits DNA methylation caused by Dnmt-1

HepG2 cells (ATCC) were plated on a 35 mm cell culture dish at 7×10⁵ cells, and cultured in DMEM (Invitrogen Corporation) added with 100 IU/mL penicillin - 100 µg/mL streptomycin solution (Invitrogen Corporation), 10% fetal bovine serum (Thermo Fisher Scientific K.K.), and a substance that inhibits DNA methylation (5-aza-2'-deoxycytidine: 5-aza (decitabine): 2.5 µM). The medium was exchanged every day. After culture for 14 days, PAS staining was performed to confirm storage capacity of glycogen which is found in mature hepatocyte (Fig. 14). The cells added with a DNA methylation inhibitor showed high glycogen storage capacity as compared to control without addition of the inhibitor.

### Example 15 Changes in expression level of hepatocyte marker in HepG2, by substance that inhibits DNA methylation by Dnmt-1

Total RNA was extracted from HepG2 cells by using miRNeasy Mini Kit (QUIAGEN). Total RNA was quantified by NanoDrop 1000 spectrophotometer (Thermo Fisher Scientific K.K.).

Total RNA sample was treated with DNase (TURBO DNA-free kit: Ambion Inc), and cDNA was synthesized from 1 µg of total RNA by using reverse transcriptase (SuperScript III Reverse Transcriptase: Ambion Inc). For quantification, SYBR Green (PlatinumSYBR Green quantitative PCR SuperMix UDG: Invitrogen Corporation) was used. The reaction for real-time PCR was performed at 95°C, 2 min, 40 cycles (95°C for 15 sec, 60°C for 30 sec). The cDNA expression level was standardized with GAPDH.

HepG2 cells were plated on a 35 mm cell culture dish at 4×10⁵ cells, and cultured in DMEM (Invitrogen Corporation) added with 100 IU/mL penicillin - 100 µg/mL streptomycin solution (Invitrogen Corporation), 10% fetal bovine serum (Thermo Fisher Scientific K.K.), and a substance that inhibits DNA methylation (5-aza-2'-deoxycytidine: 5-aza (decitabine): 2.5 µM). The medium was exchanged every day. After culture for 10 days, total RNA was extracted by the above-mentioned method, and the expression levels of genes of albumin, glucose-6-phosphatase (G6pc), tyrosine amino group transferase (Tat), and drug-metabolizing enzymes (Cyp17a1, Cyp2C19, CYP3A4, and CYP1A2), which are hepatocyte markers, were quantified by the real-time PCR method.

As shown in Fig. 15, the expression of any hepatocyte marker showed a high level when differentiation was induced in a medium containing a DNA methylation inhibitor, as compared to differentiation induction in a medium without addition of the inhibitor (control).

### Example 16 Changes in CYP enzyme activity of hepatocyte due to substance that inhibits DNA methylation caused by Dnmt-1 and Dnmt-1 inhibitory nucleic acid

HepG2 cells were plated on a 12 well plate at 3×10⁵ cells, and cultured in DMEM (Invitrogen Corporation) added with 100 IU/mL penicillin - 100 µg/mL streptomycin solution (Invitrogen Corporation), 10% fetal bovine serum (Thermo Fisher Scientific K.K.), and a substance that inhibits DNA methylation (5-aza-2'-deoxycytidine: 5-aza (decitabine): 2.5 µM, 5 µM, 7.5 µM, 10 µM, or 20 µM, or RG108: 20 µM or 100 µM). The medium was exchanged every day. On day 13 of culture, CYP activity was evaluated using P450-Glo CYP450 Assay System (Promega). As for siRNA (siDnmt-1) or miR-148a precursor that suppresses expression of Dnmt-1, CYP activity was evaluated by adding 20 nM of siRNA or miR-148a precursor, and 5 µL of a transfectin reagent (Bio-Rad) to 1 mL of OptiMEM (Invitrogen Corporation) after 72 hr of culture.

After CYP activity measurement, the medium was exchanged with DMEM (Invitrogen Corporation) added with 100 IU/mL penicillin - 100 µg/mL streptomycin solution (Invitrogen Corporation), and 10% fetal bovine serum (Thermo Fisher Scientific K.K.), and Cell Counting Kit-8 (DOJINDO LABORATORIES) was added. The absorbance (450 nm) was measured 2 hr later, and the CYP activity was normalized with the cell number.

The cells treated with a substance that inhibits DNA methylation caused by Dnmt-1 or Dnmt-1 inhibitory nucleic acid showed high CYP enzyme activity as compared to cells without the treatment (control) (Fig. 16).

### Example 17 CYP enzyme induction capacity of hepatocyte treated with substance that inhibits DNA methylation caused by Dnmt-1

HepG2 cells were plated on a 35 mm cell culture dish at 1×10⁶ cells, and cultured in DMEM (Invitrogen Corporation) added with 100 IU/mL penicillin - 100 µg/mL streptomycin solution (Invitrogen Corporation), 10% fetal bovine serum (Thermo Fisher Scientific K.K.), and a substance that inhibits DNA methylation (5-aza-2'-deoxycytidine: 5-aza (decitabine): 2.5 µM). The medium was exchanged every day. Dexamethasone (25 µM) was added after 1 week of culture. After 72 hr of culture, CYP activity induction capacity was evaluated using P450-Glo CYP450 Assay System (Promega) (Fig. 17).

When enzyme induction was performed by adding dexamethasone to hepatocyte treated with a medium containing a substance that inhibits DNA methylation caused by Dnmt-1, high enzyme induction capacity of CYP1A2 and CYP3A4 was observed as compared to differentiation induction in a medium without addition of the inhibitor (control).

From the above results, it was shown that hepatoblastoma treated with a substance that inhibits DNA methylation caused by Dnmt-1 or Dnmt-1 inhibitory nucleic acid differentiates into hepatocyte with higher maturity.

### Industrial Applicability

According to the present invention, hepatocyte (preferably hepatocyte with high maturity) can be provided in a short period, which enables stable supply of hepatocytes usable for experiments for the prediction of hepatotoxicity and liver metabolite and the like. Using hepatic progenitor cell induced from stem cell having superior proliferation capacity in the method of the present invention, hepatocytes with less lot-to-lot variation than primary cultured hepatocytes can be produced stably. Therefore, hepatocytes suitable for continuous use in drug screening and the like can be provided.

This application is based on Japanese patent application Nos. 2012-177171 (filing date: August 9, 2012) and 2013-003365 (filing date: January 11, 2013), the contents of which are encompassed in full herein.

## Claims

1. A promoter of differentiation from hepatic progenitor cell into hepatocyte, which comprises a substance that suppresses expression of Dnmt-1 or a substance that inhibits function of Dnmt-1 as an active ingredient.

2. The differentiation promoter according to claim 1, wherein the substance that suppresses expression of Dnmt-1 is a nucleic acid that suppresses expression of Dnmt-1 or a precursor thereof.

3. The differentiation promoter according to claim 2, wherein the nucleic acid that suppresses expression of Dnmt-1 or precursor thereof is miRNA for mRNA of Dnmt-1 gene or a precursor thereof, or siRNA for mRNA of Dnmt-1 gene or a precursor thereof.

4. The differentiation promoter according to claim 3, wherein the nucleic acid that suppresses expression of Dnmt-1 or precursor thereof is miR-148a or a precursor thereof.

5. The differentiation promoter according to claim 1, wherein the substance that inhibits function of Dnmt-1 is a substance that inhibits DNA methylation caused by Dnmt-1.

6. The differentiation promoter according to claim 1, wherein the substance that inhibits DNA methylation caused by Dnmt-1 is a cytidine analog or a salt, solvate, hydrate, precursor or derivative thereof.

7. The differentiation promoter according to claim 5, wherein the substance that inhibits DNA methylation caused by Dnmt-1 masks a Dnmt-1 target sequence.

8. The differentiation promoter according to claim 5, wherein the substance that inhibits DNA methylation caused by Dnmt-1 blocks an active site of Dnmt-1.

9. The differentiation promoter according to any one of claims 1 to 8, wherein the hepatic progenitor cell is a primary cultured cell or a cell induced to differentiate from a stem cell.

10. The differentiation promoter according to claim 9, wherein the hepatic progenitor cell is a primary cultured cell.

11. The differentiation promoter according to claim 9, wherein the hepatic progenitor cell is a cell induced to differentiate from an embryonic stem cell, an artificial pluripotent stem cell or a mesenchymal stem cell.

12. A method of producing a hepatocyte from a hepatic progenitor cell, comprising the following steps:
(1) a step of suppressing expression of Dnmt-1 or inhibiting function of Dnmt-1 in a hepatic progenitor cell by contacting the hepatic progenitor cell with the substance that suppresses expression of Dnmt-1 or substance that inhibits function of Dnmt-1 according to any one of claims 1 to 9; and
(2) a step of inducing differentiation into a hepatocyte by culturing the hepatic progenitor cell obtained in said step (1), wherein expression of Dnmt-1 is suppressed or function of Dnmt-1 is inhibited.

13. The method according to claim 12, wherein the hepatic progenitor cell is a primary cultured cell a cell induced to differentiate from or a stem cell.

14. The method according to claim 13, wherein the hepatic progenitor cell is a primary cultured cell.

15. The method according to claim 13, wherein the hepatic progenitor cell is a cell induced to differentiate from an embryonic stem cell, an artificial pluripotent stem cell or a mesenchymal stem cell.

16. A hepatocyte produced by the method according to any one of claims 12 to 15.

17. A method of evaluating metabolism of a test compound, comprising the steps of the following (a) and (b):
(a) a step of contacting the hepatocyte according to claim 16 with a test compound; and
(b) a step of measuring the metabolism of the test compound contacted with the hepatocyte in said step (a).

18. A method of evaluating hepatotoxicity of a test compound, comprising the steps of the following (a) and (b):
(a) a step of contacting the hepatocyte according to claim 16 with a test compound; and
(b) a step of measuring the level of disorder of the hepatocyte contacted with the test compound in said step (a).

19. A promoter of differentiation from hepatoblastoma into hepatocyte, which comprises a substance that suppresses expression of Dnmt-1 or a substance that inhibits function of Dnmt-1 as an active ingredient.

20. A method of producing a hepatocyte from a hepatoblastoma, comprising the following steps:
(1) a step of suppressing expression of Dnmt-1 or inhibiting function of Dnmt-1 in a hepatoblastoma by contacting the hepatoblastoma with the substance that suppresses expression of Dnmt-1 or substance that inhibits function of Dnmt-1 according to any one of claims 1 to 9; and
(2) a step of inducing differentiation into a hepatocyte by culturing the hepatoblastoma obtained in said step (1), wherein expression of Dnmt-1 is suppressed or function of Dnmt-1 is inhibited for not less than 7 days.
